# EUROPEAN PATENT APPLICATION

(11) **EP 1 900 819 A1**
(43) Date of publication of application: **19.03.2008**
(21) Application number: 06120714.8
(22) Date of filing: 15.09.2006
(51) Int. Cl.: C12N 15/85

(54) **Animal model for the selection and validation of agents active against pulmonary emphysema and colorectal cancer**

(71) Applicant: Frankgen Biotechnologie AG, D-61476 Kronberg (DE)
(72) Inventor: Melchner, Harald von, 61476 Kronberg (DE); Wempe, Frank, 63165 Mühlheim/Main (DE); De-Zolt, Silke, 63517 Rodenbach (DE)
(74) Representative: Helbing, Jörg

(57) **Abstract**

The present invention relates to a non-human animal model, cell and tissue cultures derived therefrom, which do not produce or produce only suboptimal levels of one or more functional sestrins and in addition do not produce or do only produce suboptimal levels of latent transforming growth factor β binding protein 4 (Itbp4). Furthermore, the present invention relates to a method for selecting agents for treating the pulmonary emphysema and/or the colorectal cancer exhibited by utilizing the animal model, cell or tissue culture of the invention. The animal model, cell or tissue culture is suitable for preclinical testing of efficacy, toxicity and bioavailability of potential agents.

## Description

The present invention relates to a non-human animal model, cell and tissue cultures derived therefrom, which do not produce or produce only suboptimal levels of one or more functional sestrins and in addition do not produce or do only produce suboptimal levels of latent transforming growth factor β binding protein 4 (Itbp4). Furthermore, the present invention relates to a method for selecting agents for treating the pulmonary emphysema and/or the colorectal cancer exhibited by utilizing the animal model, cell or tissue culture of the invention. The animal model, cell or tissue culture is suitable for preclinical testing of efficacy, toxicity and bioavailability of potential agents.

### Background of the Invention

Chronic obstructive lung disease with pulmonary emphysema (COPD) is a highly prevalent disease that has a large impact on quality of life for patients and their families and kills millions of people worldwide. While the major risk factor for COPD is the inhalation of toxic gases and particles that are primarily but not exclusively generated in tobacco smoke, primary (genetic) airway abnormalities are also involved as only a fraction of smokers develop COPD. COPD is associated with major healthcare costs, largely due to expensive treatments such as long-term oxygen therapy and hospital admissions, as well as indirect costs including loss of working capacity. Moreover, treatment of COPD is limited to symptoms as no pharmacologic intervention has been shown so far to modify the natural history of the disease (Vestbo J. and Hogg, J. C. Thorax. 61:86-8, 2006; Fabbri L. M., et al. Am J Respir Crit Care Med. 173:1056-65, 2006).

Colorectal cancer is one of the most common neoplasms in the Western hemisphere and represents a major public health challenge despite progress in detection and therapy. The overall mortality at 5 years is about 40%. While early stage disease can be cured by surgery, only palliative treatment is available for patients with non-resectable, metastatic cancer whose survival rate is between 7 - 24 months (Shaheen N. J., et al. Am J Gastroenterol. 2006).

From the above in becomes apparent that new pharmaceutical compounds for the treatment of both conditions are highly desirable and could be identified with the animal model of the invention, which exhibits both pulmonary emphysema and colorectal cancer as a result of loss of function mutations in the sestrin 2- and the latent transforming growth factor beta binding protein 4 genes.

Transforming growth factor-βs (TGF-βs) belong to a protein superfamily whose members control cell growth and differentiation in a variety of adult tissues and are involved in a wide range of immune and inflammatory responses (Shi Y. and Massague, J. Cell. 113:685-700, 2003; Sheppard D. Proc Am Thorac Soc. 3:413-7, 2006). Most cells secrete TGF-β as a functionally inactive, latent cytokine complexed to its latency associated propeptide (LAP), which interacts with its receptors only after activation by proteolytic cleavage. There are two known, structurally different latent complexes of TGF-β. The small latent complex consists of a mature TGF-β dimer bound to the N-terminal portion of the TGF-β-LAP propeptide. The large latent complex contains, in addition to TGF-β-LAP, one of the three latent TGF-β binding proteins (ltbps 1, 3 and 4). Unless displaced from the ltbps after secretion, TGF-β is usually deposited in the extracellular matrix (ECM) as a high molecular weight complex with ltbp and LAP (Annes J. P., et al. J Cell Sci. 116:217-24, 2003). All 4 existing isoforms of ltbp (ltbp 1-4) have been mutationally inactivated in the mouse (Dabovic B., et al. J Cell Biol. 56:227-232, 2002; Sterner-Kock A., et al. Genes Dev. 16:2264-2273, 2002; Shipley J. M., et al. Mol Cell Biol. 20:4879-87, 2000). While the phenotypes of the knock out mice varied among isoforms, both Itbp3 and Itbp4 mutants develop pulmonary emphysema due to defects in TGF-β signaling. However, in contrast to the emphysema developing mutant Itbp3 mice, which is relatively mild and strictly developmental (Dabovic B., et al. J Cell Biol. 56:227-232, 2002), emphysemas in mutant Itbp4 *(Itbp4*^{*-*/}*⁻)* mice worsen with age and progressively acquire the features of late onset emphysemas that develop in the context of COPD in humans (Sterner-Kock A., et al. Genes Dev. 16:2264-2273, 2002; WO 03/015505). The potential relevance of Itbp4 in the pathogenesis of COPD has been recently underscored by a clinical study reporting a highly significant correlation between specific COPD symptoms and a single nucleotide polymorphism (SNP) in *ltbp4* (Hersh C. P., et al. Am J Respir Crit Care Med. 173:977-84, 2006). In addition to the emphysema, mutant *ltbp4* mice develop invasive colorectal cancer (Sterner-Kock A., et al. Genes Dev. 16:2264-2273, 2002; WO 03/015505). A link between *ltbp4* expression and cancer progression has also been recently reported in a clinical study of patients with colon cancer (Bertucci F., et al. Oncogene. 23:1377-91, 2004).

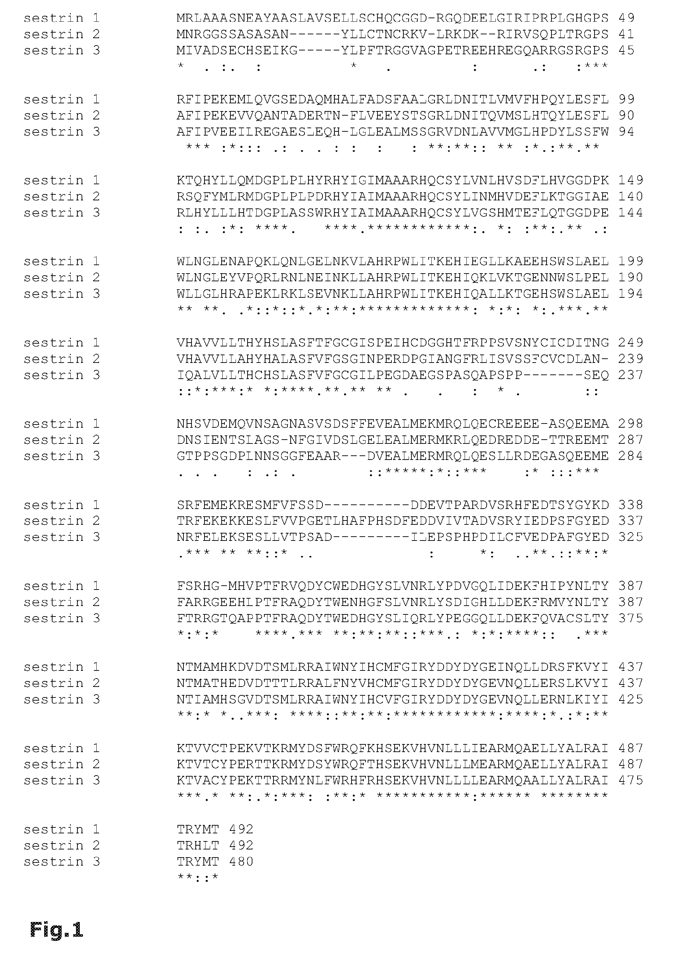

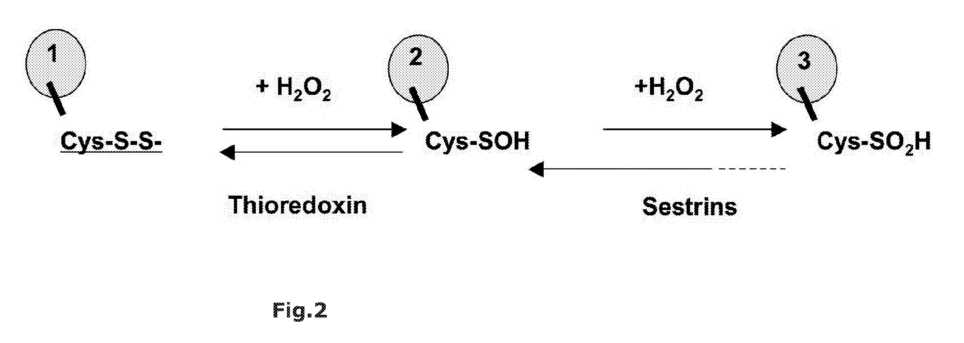

Sestrin 2 belongs to a family of highly conserved proteins initially discovered as p53 inducible proteins (Velasco-Miguel S., et al. Oncogene. 18:127-37, 1999; Peeters H., et al. Hum Genet. 112:573-80, 2003; Budanov A. V., et al. Oncogene. 21:6017-31, 2002; WO 00/12525; US 2002/0103353). Mammalian cells express three isoforms referred to as sestrin 1 (sestrin 1; also known as PA26), sestrin 2 (sestrin 2; also known as Hi95) and sestrin 3 (Figure 1). Two of the sestrins (sestrin 1 and sestrin 2) have been recently shown to regulate intracellular peroxide (ROS) levels (Budanov A. V., et al. Science. 304:596-600, 2004; Sablina A. A., et al. Nat Med. 11:1306-13, 2005). More specifically, sestrins are believed to regenerate (reduce) peroxiredoxins (Prxs), which are highly conserved and ubiquitously expressed, antioxidant proteins (reviewed in Georgiou G. and Masip, L. Science. 300:592-4, 2003; Wood Z. A., et al. Trends Biochem Sci. 28:32-40, 2003). While reducing H₂O₂ to H₂O, Prxs are oxidized at their so called peroxidatic cystein (Cys-SH), which is converted into sulfenic acid (Cys-SOH) (Figure 2). In bacteria, where the Prx AhpC is the primary H₂O₂ scavenger, the oxidized AhpC is subsequently reduced by the dedicated reductase AhpF. However, unlike AhpC, mammalian Prxs are highly susceptible to overoxidation yielding sulfinic acid (Cys-SO₂H) in presence of high peroxide concentrations (Figure 2). Protein sulfinic acids cannot be reduced by typical cellular reductants such as glutathione and thioredoxin and therefore their formation has been considered an irreversible process. Once Prx has been converted to sulfinic acid it is no longer enzymatically active and inactive enzyme accumulates in cells exposed to oxidative stress (Wood Z. A., et al. Trends Biochem Sci. 28:32-40, 2003). However, more recently gradual recovery of Prxs has been observed after initial oxidative inactivation in eukaryotic cells (Mitsumoto A., et al. Free Radic Biol Med. 30:625-35, 2001; Woo H. A., et al. Science. 300:653-6, 2003) and sestrins have been proposed as the catalyzing enzymes due to their homology to the bacterial sestrin homolog -AhpD-, which regenerates the AhpC Prx and due to their ability to reduce intracellular ROS levels (Budanov A. V., et al. Science. 304:596-600, 2004).

In eukaryotic cells Prxs are believed to be both antioxidants protecting against stress and regulators of ROS-mediated signaling (Wood Z. A., et al. Science. 300:650-3, 2003) It has been known for some time that receptor/ligand interactions generate bursts of ROS, which act as second messengers in signal transduction pathways, including the TGF-β pathway (Bae Y. S., et al. J Biol Chem. 272:217-21, 1997; Lo Y. Y. and Cruz, T. F. J Biol Chem. 270:11727-30, 1995; Mills E. M., et al. J Biol Chem. 273:22165-8, 1998; Sundaresan M., et al. Science. 270:296-9, 1995; Thannickal V. J., et al. J Biol Chem. 273:23611-5, 1998; Thannickal V. J., et al. Faseb J. 14:1741-8, 2000). Defective TGF-β signaling has been shown to be involved in the pathology of pulmonary emphysema and colorectal cancer in both animal models (including WO 03/015505 A3) and men (Morris D. G., et al. Nature. 422:169-73, 2003; Neptune E. R., et al. Nat Genet. 33:407-11, 2003; Sterner-Kock A., et al. Genes Dev. 16:2264-2273, 2002; Massague J., et al. Cell. 103:295-309, 2000; Zhu Y., et al. Cell. 94:703-14, 1998).

The association between ROS and TGF- β is complex and occurs at different levels of the signaling cascade. First, ROS have been shown to activate latent TGF-β1 both in vitro and in vivo (Annes J. P., et al. J Cell Sci. 116:217-24, 2003; Barcellos-Hoff M. H. and Dix, T. A. Mol Endocrinol. 10:1077-83, 1996). In line with this, Fatma et al. have recently reported that lens epithelial cells derived from Prx6 knock out mice are highly susceptible to oxidative stress and develop a phenotype indistinguishable from TGF-β stimulation. They could attribute this phenotype to a ROS mediated activation of latent TGF-β, which was readily reversible by antioxidants (Fatma N., et al. Cell Death Differ. 12:734-50, 2005). Second, TGF-β requires ROS for the induction of various target genes such as PAI-1, CTGF and extracellular matrix genes (Jiang Z., et al. Biochem Biophys Res Commun. 309:961-6, 2003; Park S. K., et al. Biochem Biophys Res Commun. 284:966-71, 2001). Recent studies have shown that TGF-β induced smad2,3 phosphorylation is also partly dependent on ROS (Cucoranu I., et al. Circ Res. 97:900-7, 2005) presumably via the inactivation of a dedicated phosphatase. It has been known for some time that phosphatases are susceptible to inactivation by ROS (Seo J. H., et al. Mol Biol Cell. 16:348-57, 2005; Chiarugi P. and Cirri, P. Trends Biochem Sci. 28:509-14, 2003) and a smad2,3 phosphatase (PPM1A) capable of terminating TGF-β signaling has been recently discovered (Lin X., et al. Cell. 125:915-28, 2006. Third, TGF-β itself induces superoxide production by activating NADPH oxidases presumably by the transcriptional upregulation of Nox4 (Sturrock A., et al. Am J Physiol Lung Cell Mol Physiol. 290:L661-L673, 2006).

### Description of the Invention

It was now found that the genetic ablation of a sestrin, notably sestrin 2, from the animal model (*Itbp4*^{*-*/*-*} mice model) described in WO 03/015505 resulted in a significant recovery of the disease conditions (see examples), indicating that this family of proteins could provide molecular targets for the treatment of COPD and colorectal cancer. It is believed that ablation of sestrin 2 in the animal model of WO 03/015505, reactivated TGF-β signaling, presumably by increasing intracellular second messenger ROS levels. This in turn significantly improved both disease conditions as illustrated by the examples, suggesting that pulmonary emphysema and colon cancer can be treated by antagonizing sestrin function. The present invention thus relates to
(1) a non-human animal model, which does not produce one or more functional sestrins or produces subotpimal levels of one or more sestrins, preferably sestrin 2 and in addition does not produce latent transforming growth factor β binding protein 4 (hereinafter "Itbp4") or produces subotpimal levels of Itbp4;
(2) a non-human animal model, which does not express one or more functional sestrins or expresses subotpimal levels of one or more sestrins;
(3) a cell or tissue culture isolated from the animal models as defined in (1) or (2) above;
(4) a method for preparing the non-human animal model of (1) above, which comprises disrupting the sestrin and/or ltbp4 gene in a germ cell of the animal model;
(5) a method for selecting an agent for treating a symptom occurring in the non-human animal model of 1 above comprising:
   (i) applying one or more agents to be tested to said animal model,
   (ii) determining whether one or more symptoms occurring in said animal model have changed as a result of application of said agent(s);
(6) a method selecting an agent that interferes with ROS production and TGF-β signaling comprising:
   (i) applying one or more agents to be tested to the to the cell or tissue culture of (3) above,
   (ii) determining whether cellular ROS levels and TGF-β signaling have changed as a result of the application of said agents or agents;
(7) a method to analyze whether cancer and/or pulmonary emphysema is caused by differential Itbp4 and sestrin gene or protein expression or expression level or by a defect in the Itbp4 and sestrin gene comprising:
   (i) characterizing the Itbp4 and sestrin gene or protein expression or expression level or Itbp4 and sestrin gene allele status of an individual having cancer or pulmonary emphysema,
   (ii) characterizing the ltbp4 and sestrin gene or protein expression or expression level or ltbp4 and sestrin gene allele status of a control individual, a difference in the ltbp4 and sestrin gene or protein expression or expression level or ltbp4 and sestrin gene allele status indicating that cancer and/or pulmonary emphysema and/or cardiomyopathy is linked to differential Itbp4 and sestrin gene or protein expression or expression level or a defect in the Itbp4 and sestrin genes;
(8) a method for diagnosing cancer and/or pulmonary emphysema and/or cardiomyopathy comprising:
   (i) characterizing the Itbp4 and sestrin gene or protein expression or expression level or Itbp4 and sestrin gene allele status of an individual,
   (ii) characterizing the Itbp4 and sestrin gene or protein expression or expression level or Itbp4 and sestrin gene allele status of a control individual, a difference in the LTBP-4 gene or protein expression or expression level or Itbp4 and sestrin gene allele status indicating the presence of cancer and/or pulmonary emphysema and/or cardiomyopathy in said individual;
(9) a kit for diagnosing pulmonary emphysema and/or cancer comprising means to detect the Itbp4 and sestrin gene or protein expression or expression level or the Itbp4 and sestrin gene allele status of an individual and a control individual; and
(10) a kit for performing the method of (6) above, said kit comprising the cell or tissue culture of (3) above.

The kits of (9) and (10) above may further contain instructions for performing the methods, in particular for detecting the ltbp4 and sestrin gene or protein expression or expression level or ltbp4 and sestrin gene allele status.

In conclusion, the present invention provides an animal model of human disease that reveals some crucial functions of the sestrin proteins. In view of these functions, the animal model of the invention can be used to develop novel treatments for pulmonary emphysema and cancer that target sestrin proteins and their role in ROS metabolism and TGF-β signaling.

### Description of the Figures

Figure 1: Amino acid sequence of the mouse sestrins and homology between isoforms. The sequences of the isoforms are further shown in SEQ ID NOs:1 to 3.
Figure 2: Oxidation of the peroxidatic cystein of peroxiredoxins (Prxs) by superoxides. Reduced Prxs form dimers via a disulfide bond (1). Oxidized Prxs form sulfenic acid (2), which is reduced by thioredoxin or glutathion in a reverse reaction (1). Overoxidized Prxs form sulfinic acid, which cannot be reduced by thioredoxin or glutathion (3). Sestrins are believed to regenerate the overoxidized Prxs. For further explanation see text.
Figure 3: pt1βgeo gene trap insertion in the last intron of the sestrin 2 gene (Ensembl Id: *ENSMUSG00000028893*) and position of the allele specific primers (arrows). The resulting fusion protein lacks 27 aa of sestrin 2.
Figure 4: Whole mount E7.5 embryo stained with X-Gal.
Figure 5: Analysis of sestrin 2 gene expression in W077E04 mutant mice. A. qRT-PCR of total RNA extracted from lungs of wild type (WT), heterozygous (+/-) and homozygous (-/-) mice. B. Western blot analysis of sestrin 2 protein expression in isolated mouse colon (lanes 1, 2) and lung (lanes 3, 4) fibroblasts using polyclonal anti-mouse sestrin 2 (PTG Inc., Chicago, IL) and anti-tubulin antibodies. Minor bands reacting with polyclonal anti-sestrin 2 antibody are most likely non-specific.
Figure 8: Tracheobronchial silicone casts of 5.5 months old wild type, single *Itbp4* and double *ltbp4*/*sesn2* mutant mice.
Figure 9: Pulmonary emphysema of age matched single *ltbp4* double *ltbp4*/*sesn2* mutant mice. HE stain at x 40 magnification.
Figure 10: Elastin and collagen content in single *Itbp4* and double *ltbp4*/*sesn2* mutant lungs. A. Elastin (black) stained with Weigert's resorcin-fuchsin at x 200 magnification. B. collagen (blue) stained with Masson's trichrome stain at x 200 magnification.
Figure 11: Tracheobronchial silicone cast of an *ltbp4*^{*-*/*-*} lung heterozygous for *sesn2* null alleles.
Figure 12: Collagen deposition in *Itbp4* mutant lungs heterozygous *(sesn*^{*+*/*-*}) and homozygous *(sesn*^{*-*/}*⁻)* for the *sesn2* null allele. Collagen (blue) stained with Masson's trichrome stain at x 200 magnification.
Figure 13: Colorectal adenoma in age matched single (*Itbp4*^{*-*/-}) and double (*Itbp4*^{*-*/}*⁻ sesn2*^{*-*/*-*}) mutant mice.
Figure 14: P-smad2 levels in the colon of age matched single (*Itbp4*^{*-*/*-*}) and double (*Itbp4*^{*-*/}*⁻sesn2*^{*-*/*-*}) mutant mice. Positive cells are reddish-brown.
Figure 15: P-smad2 levels in *sesn2* mutant lungs. Lung section were stained with polyclonal anti-P-smad2 antibody. Positive cells are reddish-brown.
Figure 16: Increased connective tissue deposition in mutant *sesn2* lungs. HE stain at x 200 (top) and x 400 (bottom) magnifications.
Figure 17: ROS levels in MLFs. A. Basal and H₂O₂-induced (200 µM, 3 hours) ROS amounts measured by FACS (530 nm) after DCF treatment. FL1-H, fluorescence intensity. B. Basal and H₂O₂ induced ROS amounts accumulating in MLFs expressed as mean intensity of cell fluorescence ± SD of the two independent experiments shown in A.

### Detailed Description of the Invention

The particular terms and abbreviations utilized to define the present invention are further defined in the following.

The terms "does not produce functional sestrins" and "does not produce functional ltbp4" denote the lack of effective sestrin and ltbp4 expression, respectively. A "lack of effective sestrin and ltbp4 expression" also includes the expression of non-functional (i.e. truncated) sestrins and ltbp4 proteins which are not exerting the function of the native protein.

The term "produces suboptimal levels of sestrins" and "produces suboptimal levels of Itbp4" encompasses the translation level of sestrin and Itbp4 proteins, which are insufficient to exert their function. Preferably, the level is reduced at least by 50%, more preferably by 70% and most preferably by 100 %.

The term "homozygous disruption" relates to an identical mutation in both alleles of a gene.

The term "heterozygous disruption" relates to a mutation in only one allele of gene. The term "mutation" refers to a change of one or more nucleotide pairs of a DNA molecule.

The term "insertion" is directed to a mutation identified by the presence of one or more additional base pairs in the DNA. The term "deletion" relates to a mutation generated by removal of a sequence of DNA (one or more base pairs), the regions on either side being joined together. The term "substitution mutation" is directed to a nucleotide exchange. The substitution mutation can result in an amino acid change or can introduce a premature translation stop codon. Furthermore, a substitution mutation can affect splicing or expression of the gene when occurring at sites necessary for splicing or gene regulation.

The term "gene targeting" relates to a type of homologous recombination that occurs when a fragment of genomic DNA is introduced into a cell and that fragment recombines with homologous sequences in the genome. The term "gene trap integration" is directed to insertion of a vector, which comprises a reporter gene and which is activated upon insertion into an active transcription unit of the genome. The term "mutagenesis" denotes a chemical or physical treatment that changes nucleotides in the genome of an organism. An example of a chemical mutagenesis is N-ethyl-N-nitrosurea (ENU) mutagenesis.

The term "exon" encompasses a segment of a gene, which is decoded to give a mRNA product. Individual exons may contain protein coding DNA and/or noncoding DNA. The term "intron" denotes non-coding DNA, which separates neighboring exons in a gene. During gene expression, introns like exons are transcribed into RNA but the transcribed intron sequences are subsequently removed by RNA splicing and are not present in mRNA. The term "regulatory region" relates to the nucleotide sequence, which comprises regions that are necessary for the regulation of gene transcription. These regions comprise, for example, promoters and enhancers and they can be located in 5' untranslated regions, exons, introns and 3'UTRs. The term "splice site" encompasses the nucleotides at the beginning and the end of the intron that are required for the joining of two exons by removing the intercalated intron during primary transcript processing to functional mRNA.

The term "pulmonary emphysema" denotes a symptom of chronic obstructive lung disease (COPD) characterized by a size increase beyond normal of air spaces distal to the terminal bronchioles and inflammatory infiltrates.

The term "cardiomyopathy" designates a primary non-inflammatory disease of the heart muscle, which is the result of pulmonary hypertension that complicates COPD. The term "cancer" refers to an uncontrolled proliferation of epithelial cells lining the colonic crypts.

"ROS metabolism" refers to the production and neutralization (reduction) of intracellular reactive oxygen species such as hydrogen peroxide and oxygen anions.

The term "profibrotic changes" refers to an increased tissue deposition of collagen associated with a multiplication of fibroblasts.

The term "selecting an agent for treating a symptom" encompasses choosing a composition for management of the condition.

The term "application of one or more agents" relates to administering single compounds or compound combinations orally, by inhalation, parenterally, e.g. intravenously, subcutaneously, intraperitoneally or intramuscularly, or topically, e.g. ophtalmically, vaginally, rectally or intranasally.

The invention is hereinafter described in more detail by referring to the accompanying Figures and Examples.

In a preferred embodiment of aspect (1) of the invention, the genome of the animal comprises a homozygous disruption of the *sesn2* and *ltbp4* genes. Preferably this homozygous disruption has been generated by a mutation and this mutation can be an insertion, deletion or a substitution mutation. Furthermore, preferably said mutation is generated by gene targeting, gene trapping or chemical mutagenesis and it has occurred in an exon, intron, regulatory region or splice site of the sestrin 2 gene, preferably into the last intron (i.w. the 9^{th} intron) of the sestrin 2 gene, and in an exon, intron, regulatory region or splice site of the Itbp4 gene, preferably in the 5^{th} intron of *Itbp4*. It is also preferred that the mutation sites rise to an expression of a detectable reporter gene such as fluorescent proteins (such as GFP and its derivatives), enzymes (such as LacZ) or selection markers (such as βgeo), or the like. Particularly preferred is that
(i) the sestrin 2 gene is disrupted in the 9^{th} intron by inserting a gene trap vector, preferably pT1βgeo (SEQ ID NO:10); and/or
(ii) the Itbp4 gene is disrupted in the 5^{th} intron by inserting a gene trap vector, preferably U3Cre (SEQ ID NO: 16).

In a further preferred embodiment said animal comprises a heterozygous disruption of the sens2 gene and a homozygous disruption of ltbp4 gene or homozygous disruption of the sestrin 2 gene and a heterozygous disruption of the ltbp4 gene.

In another aspect of the invention the animal model exhibits pulmonary emphysema and/or cardiomyopathy and/or colorectal cancer. Furthermore, the non-human animal is preferably a non-human mammal, more preferably a rodent including mouse and rat.

In a further preferred embodiment the animal model exhibits defects in ROS metabolism and/or profibrotic changes in one or more major organs, peferably in the lung or colon.

In a further preferred embodiment the animal model develops symptoms that are less severe than those exhibited by the animal model disclosed in WO 03/015505 A3.

Embodiment (3) of the invention relates to a cell or tissue culture isolated from a non-human animal model (1) or (2). Preferably, the cell is derived from the lung or colon.

In a preferred aspect of this embodiment, the cell or tissue culture is isolated from a non-human animal model whose genome comprises a homozygous disruption of one or more sestrin genes such that said genes do not produce functional sestrins, preferably sestrin 2 and a homozygous disruption of the ltbp4 gene such that said gene does not produce functional ltbp4.

In another preferred aspect of this embodiment, the cell or tissue culture is isolated from a non-human animal model whose genome comprises a heterozygous disruption of one or more sestrin genes such that said genes do produce only 50% or less of functional sestrins, preferably sestrin 2 and a homozygous disruption of the Itbp4 gene such that said gene does not produce functional Itbp4.

In another preferred aspect of this embodiment, the cell or tissue culture is isolated from a non-human animal model whose genome comprises a homozygous disruption of one or more sestrin genes, preferably sestrin 2 and a heterozygous disruption of *ltbp4* such that said gene produces only 50% or less of functional Itbp4. Particularly preferred is that the cell or tissue culture is derived from lung or colon.

Embodiment (4) of the invention relates to a method for preparing the non-human animal model of embodiments (1) and (2), which comprises disrupting the sestrin and/or ltbp4 gene in a germ cell of the animal model. In a preferred aspect of this embodiment, the non-human animal is a non-human mammal and the germ cell is an ES cell

Embodiment (5) of the invention is directed to a method for selecting an agent or agents for treating a symptom occurring in the animal model of the invention comprising: (i) applying one or more agents to be tested to the animal model of the invention; and (ii) determining whether one or more symptoms occurring in the animal model of the present invention have changed as a result of the application of said agent or agents. In a preferred embodiment the symptom is selected from a group consisting of pulmonary emphysema, cardiomyopathy and cancer. In a further aspect of the invention the agent, which is suitable for treating a symptom occurring in the animal model of the invention is a pharmaceutical. Furthermore, the present invention relates to the use of the agent, which is suitable for treating a symptom occurring in the animal model of the invention for the preparation of a pharmaceutical composition for the treatment of pulmonary emphysema. Moreover, the present invention is directed to a method of treatment of cancer and pulmonary emphysema using the agent, which is suitable for treating a symptom occurring in the animal model of the present invention.

Embodiment (6) of the invention is directed to a method selecting an agent that interferes with ROS production and TGF-β signaling comprising:
(i) applying one or more agents to be tested to the to the cell or tissue culture of (3) above,
(ii) determining whether cellular ROS levels and TGF-β signaling have changed as a result of the application of said agents or agents.

It is particularly preferred in said method that the cell or tissue culture is from lung or colon.

Embodiment (7) of the invention is a method to analyze whether pulmonary emphysema and/or cancer is caused by differential Itbp4 and sestrin gene or protein expression or expression level or by a defect in the Itbp4 and sestrin genes comprising the (i) characterization of the Itbp4 and sestrin gene or protein expression or expression level or of the Itbp4 and sestrin gene allele status of an individual having pulmonary emphysema and/or cancer, and the (ii) characterization of the Itbp4 and sestrin gene or protein expression or expression level or Itbp4 and sestrin gene allele status of a control individual. A difference in the Itbp4 and sestrin gene or protein expression or expression level or in the Itbp4 and sestrin gene allele status indicates that a defect in the Itbp4 and sestrin genes is involved in the pathogenesis of pulmonary emphysema and/or cancer.

Embodiment (8) of the invention relates to a method for diagnosing pulmonary emphysema or cancer comprising the (i) characterization the Itbp4 and sestrin gene or protein expression or expression level or the Itbp4 and sestrin gene allele status of an individual and (ii) characterization of the Itbp4 gene or protein expression or expression level or Itbp4 and sestrin gene allele status of a control individual. A difference in the Itbp4 and sestrin gene or protein expression or expression level or in the Itbp4 and sestrin gene allele status would indicate the presence of pulmonary emphysema and/or cancer in said individual.

"Individual" used in connection with the embodiments (7) and (8) relates to an individual with suspected abnormal Itbp4 and sestrin gene allele status, i.e. a patient. "Control individual" refers to a healthy individual having normal Itbp4 and sestrin allele status.

In a preferred embodiment of the methods to analyze whether pulmonary emphysema and/or cancer are linked to Itbp4 and sestrin and for diagnosing pulmonary emphysema and cancer, the ltbp4 and sestrin gene allele status and the ltbp4 and sestrin expression or expression level are detected by genomic PCR, RT-PCR, Northern analysis, microarray (DNA chip) analysis or antibodies directed to the Itbp4 and sestrin proteins.

The methods of embodiments (5) to (8) are suitable to be performed *in vivo* and *in vitro.*

The animal model of the present invention can be used to dissect the molecular mechanisms controlling the sestrin/TGF-β pathway and for the identification and cloning of modifier genes able to modify, aggravate, reduce or inhibit the phenotype associated with pulmonary emphysema and cancer or other conditions occurring in the animal model of the invention. Moreover, the animal model can be used for identification of early diagnostic markers for cancer and/or pulmonary emphysema or other conditions occurring in the animal model of the invention. In addition, the animal model of the present invention can be used for the monitoring of the activity of agents useful in the prevention or treatment of cancer and/or pulmonary emphysema or other conditions occurring in the animal model of the invention and as a test model system for agents suspected of promoting or aggravating cancer and/or pulmonary emphysema or other conditions occurring in the animal model of the invention.

The invention furthermore explained in the following examples which are, however, not to be construed as a limitation of the invention.

### Examples

### Methods

Cell cultures: [129/SvPas] strain derived ES cells were grown on irradiated (32 Gy) MEF feeder layers in DMEM supplemented with 15% (v/v) preselected and heat inactivated fetal calf serum (FCS) (Linaris, Bettingen, Germany), 100 mM nonessential amino acids (Gibco), 0.1 mM mercaptoethanol (Sigma), 1000 U/ml of leukemia inhibitory factor (LIF) (Esgro^{R}; Gibco /BRL), as described (De-Zolt S., et al. Nucleic Acids Res. 34:e25, 2006). Lung and colon fibroblast cultures from adult wild-type and sens-/- mice were established according to standard protocols as previously described (Koli K., et al. J Cell Biol. 167:123-33, 2004) and grown in Dulbeccos's modified Eagle's medium (DMEM) supplemented with 10% fetal calf serum (Life Technologies), 100 IU/ml penicillin and 50 µg/ml streptomycin. During the first two weeks in culture spontaneous immortalization occurred generating cell lines.

5' RACE and sequencing: cDNAs from gene trap expressing ES cell lines were prepared from the polyadenylated RNA using a RoboAmp robotic device (MWG Biotech, Ebersberg, Germany) with a processing capacity of 96 samples/day. Samples of 2×10⁵ cells were lysed in 1 ml of lysis buffer containing 100 mM Tris/HCl pH 8.0, 500 mM LiCl, 10 mM EDTA, 1% LiDS and 5 mM DTT. Polyadenylated RNA was captured from the lysates by biotin-labeled oligo-d(T)-primers according to the manufacturers instructions (Roche Diagnostics Corp., Indianapolis, IN, USA) and placed on streptavidin-coated 96-well plates (AB Gene, Surrey, UK). After washing, solid-phase cDNA synthesis was performed in-situ using random hexamers and SuperScript II RT (Invitrogen, Karlsruhe, Germany). To remove excess primers the cDNAs were filtered through Multiscreen PCR plates (Millipore Corp. Bedford, MA, USA). The 5' ends of the purified cDNAs were tailed with dCTPs using terminal transferase -TdT- (Invitrogen, Karlsruhe, Germany) following the manufacturer's instructions. For PCR amplification of the cellular sequences appended to the gene trap transcripts the following vector-specific primers were used: 5'-CTA CTA CTA CTA GGC CAC GCG TCG ACT AGT ACG GGI IGG GII GGG IIG-3' (SEQ ID NO:4) and 5'-GCC AGG GTT TTC CCA GTC ACG A-3' (SEQ ID NO:5);and for nested PCR: 5'-CTA CTA CTA CTA GGC CAC GCG TCG ACT AGT AC-3' (SEQ ID NO:6) and 5'-TGT AAA ACG ACG GCC AGT GTG AAG GCT GTG CGA GGC CG-3' (SEQ ID NO:7). Amplification products were directly sequenced using AB377 or ABI3700 sequencing machines (Applied Biosystems ABI, Foster City, USA).ES cell injections, breeding and genotyping: W077E06 (TBV-2; 129SvPas) ES cell derived chimeras were generated by injecting C57BI/6 blastocysts. The resulting male chimeras were bred to C57BI/6 females, and F1 agouti offspring containing the disrupted transgene were intercrossed to obtain homozygous F2 mice. Genotyping was performed on mouse tail DNA by genomic PCR using primers against the sequences flanking the gene trap insertion previously identified in ES cells by inverse PCR and sequencing.

Histology, histochemistry and immunohistochemistry: Paraffin sections of mouse tissues were prepared and stained using standard histology procedures. To visualize elastin and collagen fibers, microscopic slides were stained with Weigert's resorcin-fuchsin or Masson's trichrome stain as previously described (Sterner-Kock A., et al. Genes Dev. 16:2264-2273, 2002). Immunostainings using rabbit polyclonal anti- P-Smad2 antibodies were performed as described in Sterner-Kock et al (Sterner-Kock A., et al. Genes Dev. 16:2264-2273, 2002)

Plastinations: Tracheas of lungs prepared from wild-type and mutant mice were injected at room temperature with 1-2 ml of E RTV silicone (Dow Corning, Midland, MI, USA) using a 2 ml syringe. Curing Agent was added at a 1:10 ratio to the silicone polymere immediately prior to injection. Injection was stopped when the silicone was visible under the surface of both lungs. Following injection, the silicone was allowed to harden fro 24-48 hours after which the specimens were placed in 10% potassium hydroxide solution for 5-7 days. and then left in boiling water for 8-12 hours to detach the tissue from the polymer. Following maceration in bioling water, the specimens were placed in 5% hydrogen peroxide for about 2 hours to complete the removal of residual tissues. Casts were then rinsed in running water overnight.

RNA isolation, RT-PCR and protein anlysis: Total cellular RNA was isolated using RNeasy Mini kit (Qiagen) according to manufacturer's instructions. RNA concentrations and purities were determined spectrophotometrically (Ultrospec 3000, Amersham) as well as by agarose gel electrophoresis followed by ethidium bromide staining. RT-PCRs were performed according to standard protocols by using 75 ng of reverse-transcribed total RNA in a total volume of 50 µl. Real-time RT-PCR analysis of sestrin 2 gene expression in ES cells was performed using SYBR green chemistry (ABgene, Epsom, UK) and an iCycler (Biorad) machine. cDNA was synthesized from total RNA using random priming and Superscript II (Invitrogen) reverse transcriptase. PCR reactions were run as triplicates in 25-µl volumes on 96-well plates, with each reaction containing cDNA derived from 15 ng of total RNA, 1x ABsolute SYBR fluorescein mix (ABGene) and 5 pmol of each of the following primers: 5'-CCTGGAACGGAACCTCAAAATC-3' (SEQ ID NO:8) and 5'-GGGCTTCAAGGAG-CAGCAAG-3'(SEQ ID NO:9). Amplification reactions were allowed to proceed for 35 cycles at 94 °C for 15 s, 61 °C for 30 s, and 72 °C for 30 s. For Western blotting lysates of lung and colon fibroblasts were resolved by SDS-PAGE, transfered to nitrocellulose membranes and reacted with rabbit anti-sestrin 2 polyclonal antibody (PTG Inc., Chicago, IL).

Determination of intracellular peroxide levels: 3 x 10⁵ lung or colon fibroblasts from wild type and *sesn2*^{*-*/*-*} mice plated into 6 cm Petri dishes a day before were trypsinized, washed in serum free DMEM and resuspended in 5 ml of serum free DMEM. To some cultures H₂O₂ was added to a final concentration of 200 µM. After incubating for 3 hours dichlorodihydrofluorescein (DCF) was added to the cells at a final concentration of 30 µM and incubated for another hour. After that cell were wahed in PBS, trypsinized and resuspended in 300 µl PBS which were subjected to FACS analysis.

### Example 1: A pt1βgeo gene trap insertion in the sestrin 2 gene induces a null mutation in transgenic mice.

The W077E06 gene trap ES cell line was obtained by electroporating the pt1βgeo gene trap vector shown in SEQ ID NO: 10 into TBV-2 (129SvPas) ES cells as previously described (Floss, T. & Wurst, W. Methods Mol Biol 185, 347-79, 2002). Gene trap expressing ES-cell clones were selected in 200 µg/ml G418 (Gibco/BRL), manually picked, expanded, and stored frozen in liquid nitrogen. The gene trap insertion in the 9^{th} intron of the sestrin 2 (*sesn2*) gene was identified among the recovered clones by 5' RACE as described in the Methods section and by sequence database analysis (Genbank at http://www.ncbi.nlm.nih.gov) using the BlastN algorithm. The gene trap insertion in the W077E06 cell line was verified by genomic PCR (see Methods) using the following primers complementary to gene trap- and adjacent upstream intron sequences: 5'-CAGCCTTGAGCCTCTGGAGC-3' (SEQ ID NO:11) and 5'-CTACCCTGAGAAGACGACCCG-3' (SEQ ID NO:12). The veryfied W077E06 ES cells were then converted into mice by blastocyst injection. F1 mice carrying the gene trap allele were intercrossed and the F2 offspring was mated to *ltbp4*^{*-*/*-*} mice (WO 03/015505 A3) to obtain double knock-out mice.

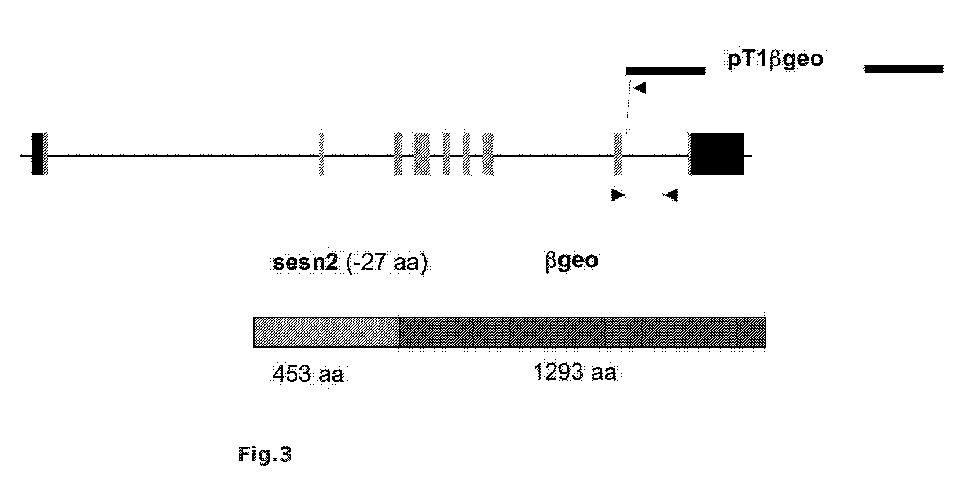

Figure 3 shows the genotyping strategy for the W077E06 (sesn2) mice. Tail DNA was PCR amplified in parallel reactions using a forward primer in the 9^{th} exon (5'-CTACCCTGAGAAGACGACCCG-3'; SEQ ID NO:13) and two reverse primers; one in the 9^{th} intron for detecting the wild type allele (5'-GGACAAATCAAGGTTACACAGAAAAAAGTC-3'; SEQ ID NO:14) and the other in the gene trap's splice acceptor site for detecting the gene trap allele (5'-CAGCCTTGAGCCTCTGGAGC-3'; SEQ ID NO:15). Amplification reactions were allowed to proceed for 30 cycles at 94 °C for 15 s, 61 °C for 30 s, and 72 °C for 30 s.

Intercrossing F1 heterozygous offspring yielded homozygous offspring at a frequency consistent with a Mendelian inheritance pattern of the disrupted transgene, indicating that sesn2 is not required for development. Mutant mice developed normally after birth and were grossly indistinguishable from their wild type and heterozygous littermates.

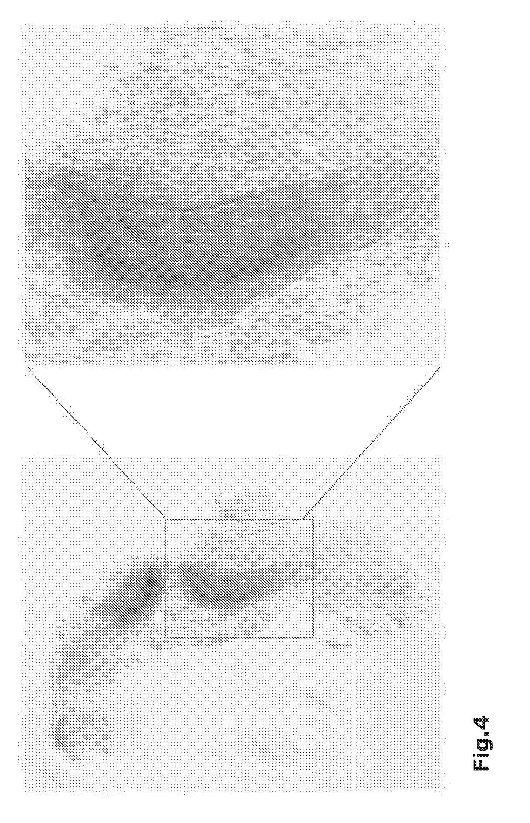

The gene trap insertion in the 9^{th} intron of the *sesn2* gene induces a fusion transcript in which the exons upstream of the insertion are spliced in frame to βgeo. Because transcription is terminated at the gene trap's polyadenylation site, the processed fusion transcript encodes a truncated version of sesn2 and the βgeo reporter (Figure 3). Figure 4 shows faint expression of this protein in an early heterozygous embryo stained with X-Gal.

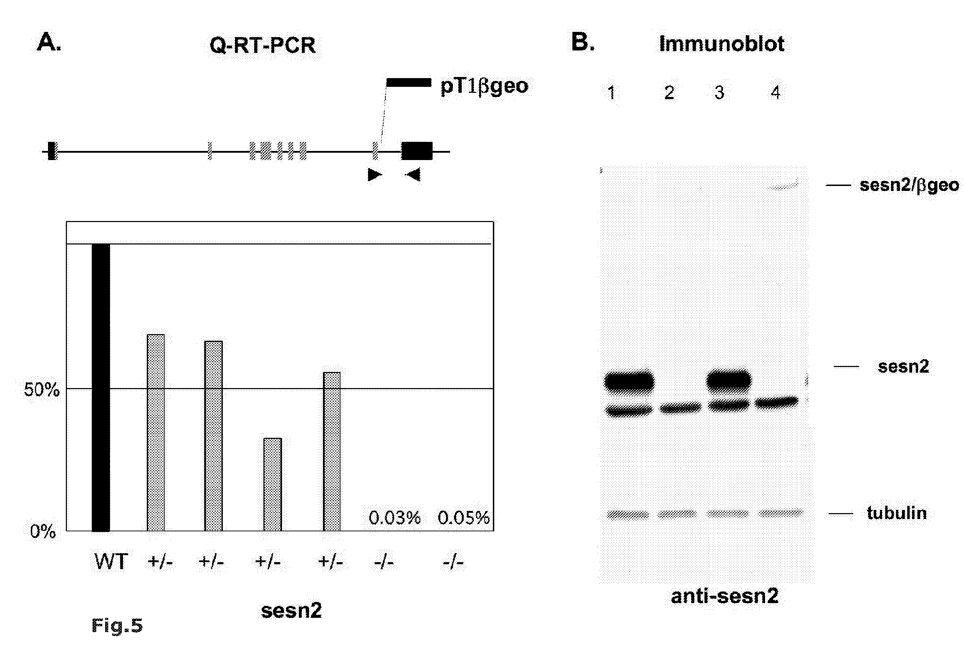

Wild type *sesn2* transcripts were completely lost from lungs of homozygous W077E06 mice whereas heterozygous lungs expressed about 50% of wild type levels (Figure 5A). Moreover, in isolated lung (MLF) and colon (CLF) fibroblasts, which both express high levels of sesn2 (Figure 5B), no protein was detected in cells from homozygous W077E06 mice, suggesting that the gene trap insertion had induced a null mutation (Figure 5B). Consistent with the faint β-galactosidase expression in the early embryos (Figure 4), mutant MLFs expressed only trace amounts of the sesn2/βgeo fusion protein, implying that the relatively large protein is unstable (Figure 5B). Given its low expression, this protein is unlikely to have a dominant negative effect even if the truncated protein retained some residual function.

### Example 2: sesn2 null alleles improve the pulmonary emphysema in ltbp4^{-/-} mice.

129/Sv (D3) ES cells with a U3Cre gene trap vector (SEQ ID NO:16) insertion in the 5^{th} intron of the ltbp4 gene were injected C57BL/6 blastocysts as previously described (WO 03/015505, Thorey, I.S. et al. Mol Cell Biol 18, 3081-3088, 1998). The resulting male chimeras were bred to C57BL/6 females and agouti offspring were tested for transgene transmission by tail blotting. Mouse tail DNA was cleaved with BglII which does not cut provirus. The DNA was fractionated on 1% agarose gels, blotted onto Hybond N nylon filters (Amersham/ Pharmacia, Piscataway, NJ) and hybridized to a ³²P labeled provirus flanking sequence probe. Animals heterozygous for the gene trap insertion were backcrossed to C57BL/6 mice for at least six generations before analyzing the phenotypes in heterozygous and homozygous offspring. To obtain double mutant strains, heterozygous *ltbp4*^{*+*/*-*} mice were crossed to homozygous *sesn2*^{*-*/*-*} mice and the offspring was genotyped by tail DNA PCR as described in Example 1 using the following allele specific primers: ltbp4 wild type allele = 5'-CCAATCTTGCTTCTTTGCTGAGC-3' (SEQ ID NO: 17) and 5'-GGC-TCATGCTTGAATGTTTCAG-3' (SEQ ID NO: 18); ltbp4 gene trap (mutant allele) = 5'-CCAATCTTGCTTCTTTGCTGAGC-3' (SEQ ID NO: 19) and 5'-ATCATGCAAGCTGGTGG-CTG-3' (SEQ ID NO:20); sesn2 wild type allele = 5'-CTACCCTGAGAAGACGACCCG-3' (SEQ ID NO:21) and 5'-GGACAAATCAAGGTTACACAGAAAAAAGTC-3' (SEQ ID NO:22); sesn2 gene trap (mutant allele) = 5'-CTACCCTGAGAAGACGACCCG-3' (SEQ ID NO:23) and 5'-CAGCCTTGAGCCTCTGGAGC-3' (SEQ ID NO:24).

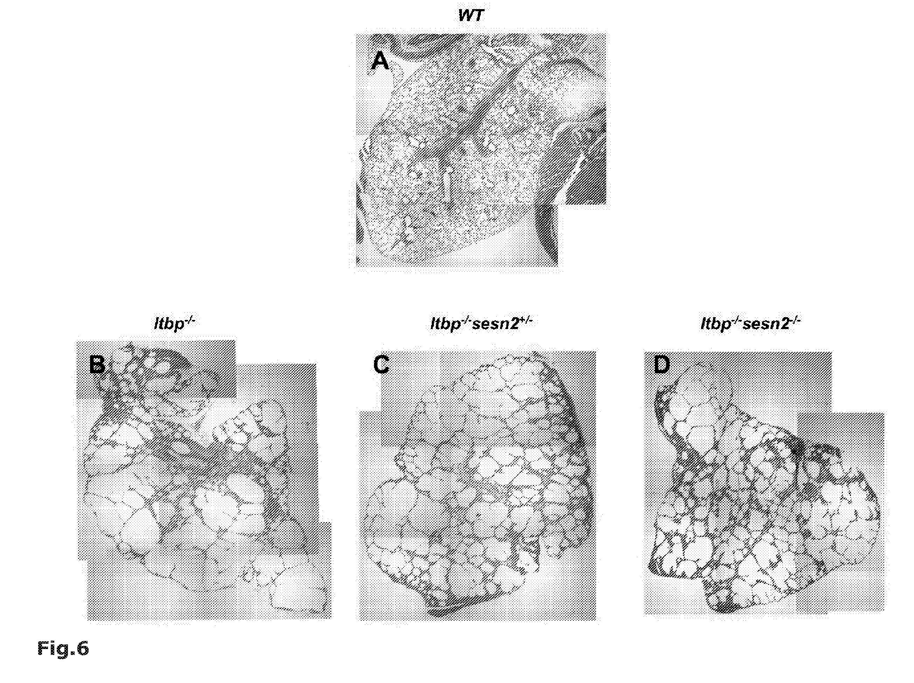

Figure 6B shows a typical emphysematous lung of an adult *ltbp4*^{*-*/*-*} mouse (WO 03/015505). It displays massively dilated alveoli surrounded by thin, dysplastic and frequently disrupted septal walls. The lobular connective tissue is significantly reduced, and lungs display multifocal atelectatic areas. Consistent with a partial recovery alveolar spaces in double mutant *ltbp4*^{*-*/}*⁻sesn*^{*-*/*-*} littermates were more numerous, reduced in size and separated by thicker walls (Figure 6D).

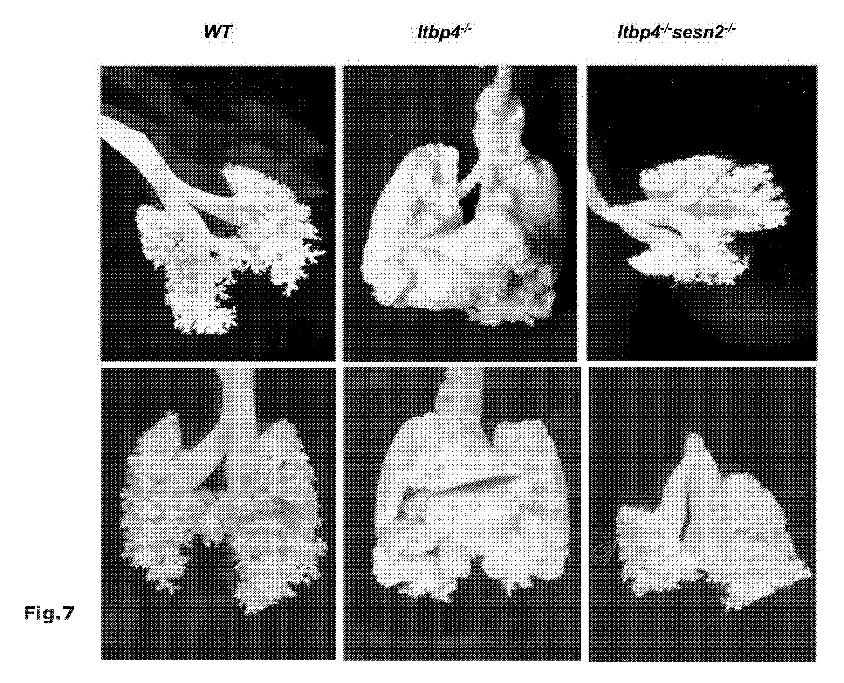

To detect the extent of damage to the bronchioles, alveolar ducts and alveoli, we visualized the tracheobronchial trees of these animals by plastination. Plastination involves the instillation of silicone into the trachea of the isolated lung. Since the alveolar ducts prevent the silicone from entering the alveolar sacs, the technique provides an informative, three dimensional image of the tracheobronchial tree (Perry S. F., et al. Exp Lung Res. 26:27-39, 2000). Figure 7 (left panels) shows the tracheobronchial tree of two 5.5 months old wild type mice with ramifications down to the terminal bronchioles and alveolar ducts. In mutant *Itbp4* littermates, these ramifications were almost completely obscured by enlarged airspaces filled with silicone, suggesting that the terminal bronchioles and alveolar ducts are enlarged and leaky (Figure 7, middle panels). However, a dramatic improvement in the tracheobronchial architecture was observed in *ltbp4*^{*-*/*-*} mice carrying two sesn2 null alleles (*ltbp4*^{*-*/}*⁻sesn2*^{*-*/*-*}) (Figure 7, right panels). In contrast to an almost invisible tracheobronchial architecture in *ltbp4*^{*-*/*-*} mice, the double mutant tracheobronchial tree was close to normal again, implying a regeneration of terminal bronchioles and alveolar ducts. These modification were much more dramatic than the parenchymal changes presumably reflecting an uneven regeneration of collagen and elastin fibers, which are the major components of the pulmonary ECM (Suki B., et al. J Appl Physiol. 98:1892-9, 2005). Although both form dense fiber networks throughout the lung, the elastin fibers are distributed evenly whereas the collagen fibers tend to condense around terminal bronchioles and alveolar ducts (Toshima M., et al. Arch Histol Cytol. 67:31-40, 2004). Based on this, we speculated that the preferential recovery of the tracheobronchial tree in the *ltbp4*^{*-*/}*⁻sesn2^{-l-}* mice could be the result of an excess collagen deposition into the pulmonary ECM.

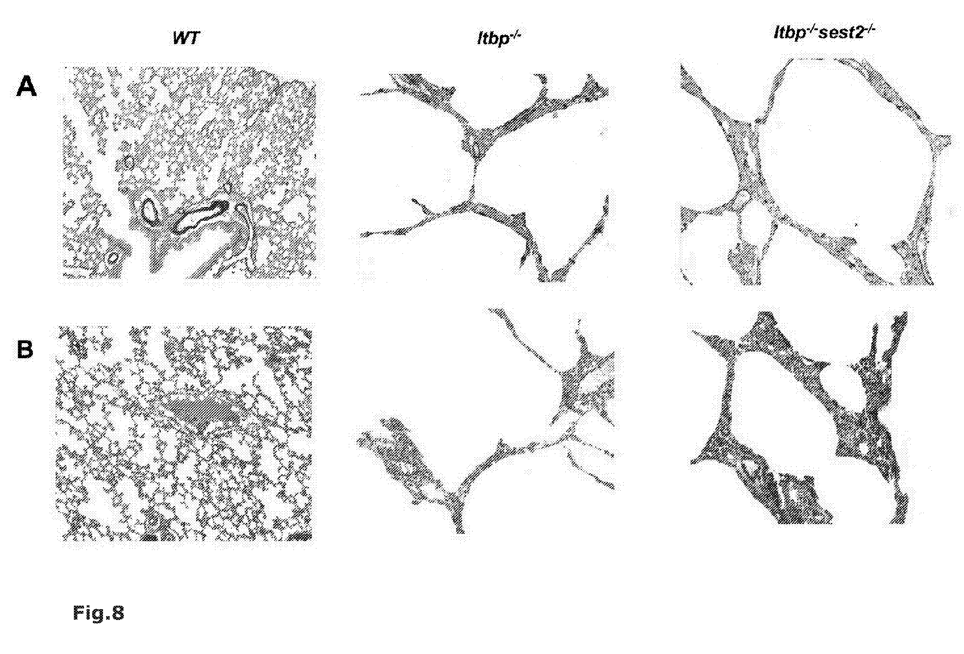

To test this, we visualized elastin and collagen in lung tissue sections using specific histochemical stains. Figure 8A shows the elastin network of *Itbp4*^{*-*/*-*} and *Itbp4*^{*-*/}*⁻ sesn2*^{*-*/*-*} lungs appearing fragmented, patchy and condensed in both. In contrast, collagen deposition was dramatically increased in the double mutant lungs (Figure 8B). As TGF-β is one of the most potent collagen inducers, the excessive deposition of collagen suggested a reactivation of TGF-β signaling in the *ltbp4*^{*-*/}*⁻sesn2*^{*-*/*-*} lungs.

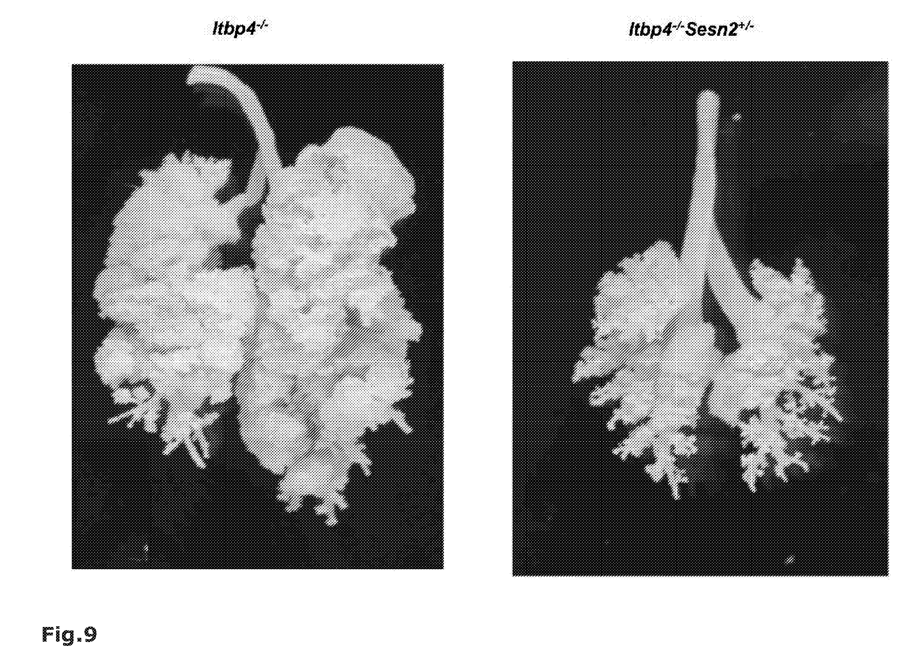

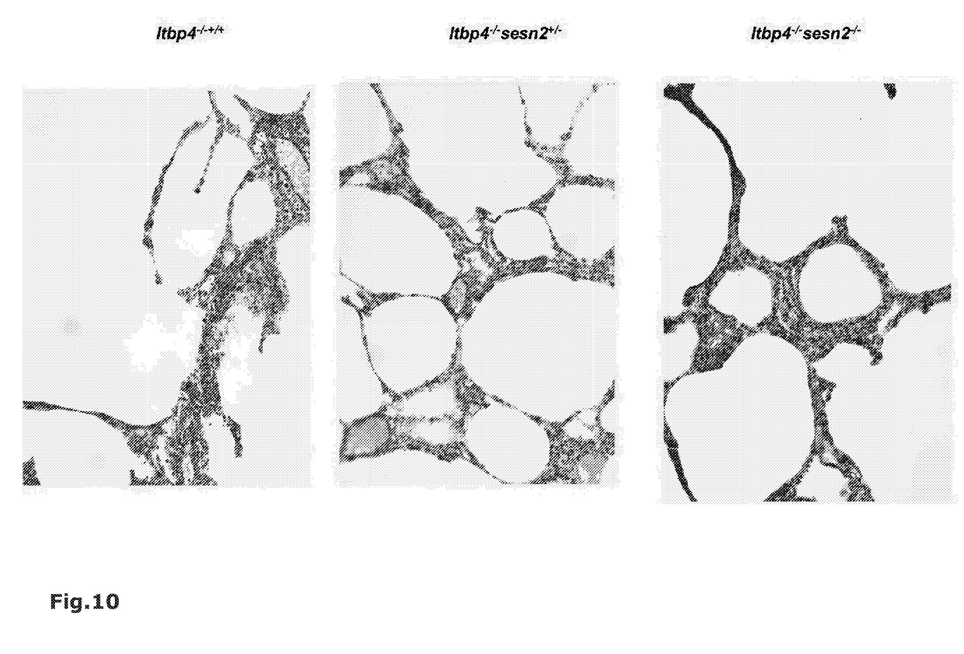

Since trancheobronchial recovery and increased deposition of collagen was also noticed in *ltbp4*^{*-*/*-*} mice heterozygous for sesn2 (*ltbp4*^{*-*/}*⁻sesn2*^{+/-}) (Figures 9, 10), the *sesn2* mutation could be haploinsufficient.

### Example 3: sesn2 null alleles improve the rectal prolapse and colorectal adenomas in ltbp4^{-/-} mice.

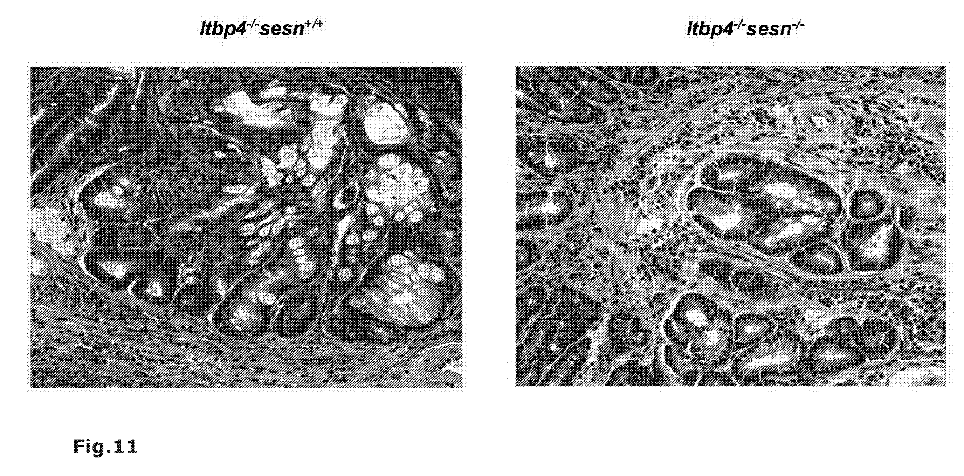

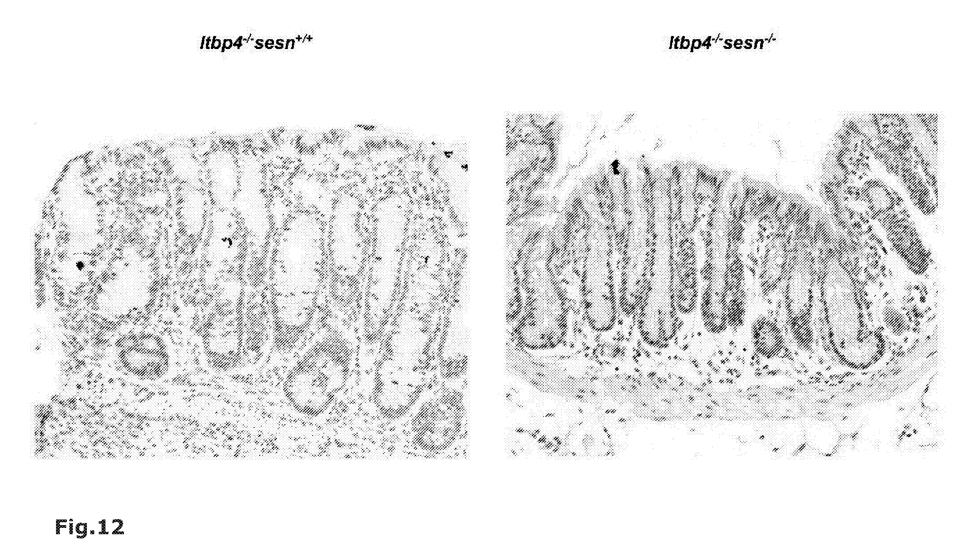

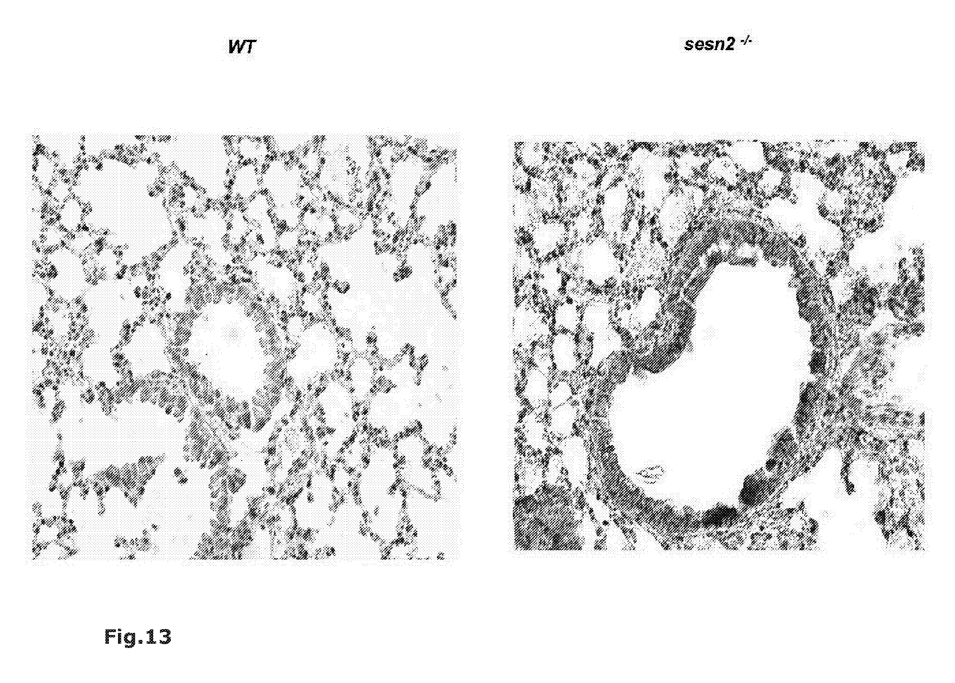

Figure 13 shows a typical colorectal an adenoma of 3 months old *lbp4*^{*-*/*-*} (WO 03/015505 A3) mouse. Microscopically, the region exhibited abberant crypt foci containing regenerating epithelial cells and an increased number of goblet cells. Although double mutant *ltbp4*^{*-*/}*⁻sesn*^{*-*/*-*} littermates also exhibited adenomas, these were sigificantly smaller in size and contained fewer goblet cells (Figure 11), suggesting a partial phenotypic rescue. Since the ltbp4-/- phenotype is essentially caused by defective TGF-β activation (Sterner-Kock A., et al. Genes Dev. 16:2264-2273, 2002), we tested wether the partial rescue was associated with a reactivation of TGF-β signaling. Towards this end we determined the levels of phosphorylated smad2 in tissue sections by immunohistochemistry. Consistent with an activation of TGF-β signaling in *ltbp4*^{*-*/}*⁻sesn2*^{*-*/*-*} mice, p-smad2 levels werevery high in contrast to the single mutant *ltbp4*^{*-*/*-*} mice, which have no detectable P-smad2 in their colon (Figure 12).

### Example 4: Sesn2^{-/-} lungs exhibit enhanced TGF-β signaling.

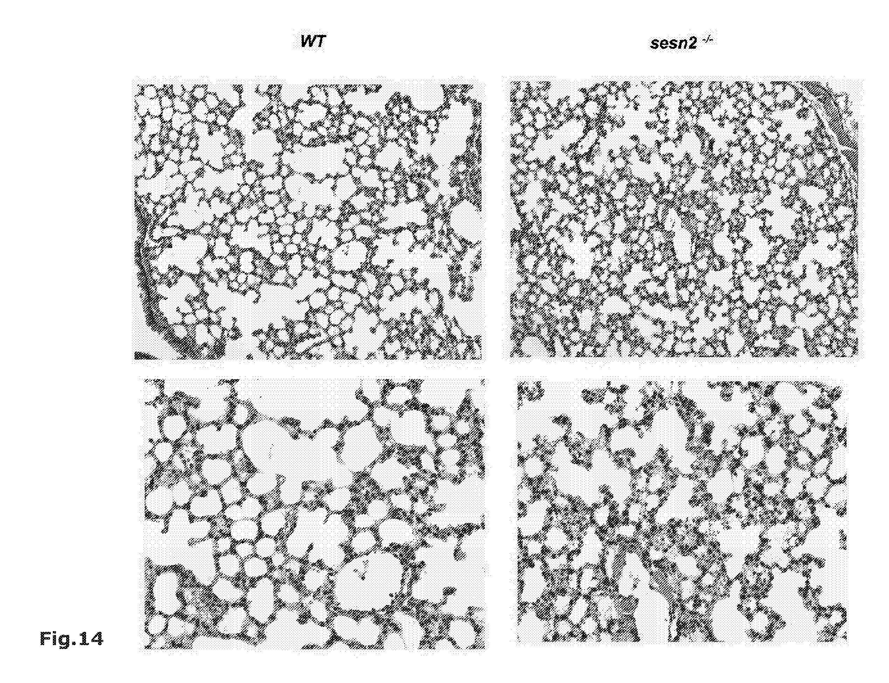

The increased collagen deposition in the lung and the increased P-smad2 levels in the colon of *ltbp4*^{*-*/}*⁻sesn2*^{*-*/*-*} mice suggested the TGF-β pathway might be similarly activated in the *sesn2*^{*-*/*-*} mice despite their grossly normal phenotype. To test this we visualized P-smad2 in lung tissue sections as described above. In a preliminary experiment, we found that P-smad2 levels in *sesn*^{*-*/*-*} lungs significantly exceeded the wild type levels, suggesting that loss of sestrin 2 activates TGF-β (Figure 13). Moreover, although we could not detect increased collagen deposition by Masson's trichrome stain (data not shown), the *sesn2*^{*-*/*-*} lungs exhibited more abundant connective tissue, smaller airspaces and thicker interalveolar walls, which are all consistent with incipient, TGF-β induced fibrosis (Figure 14) Sime P. J., et al. J Clin Invest. 100:768-76, 1997; Lee M. S., et al. Am J Pathol. 147:42-52, 1995; Sanderson N., et al. Proc Natl Acad Sci U S A. 92:2572-6, 1995).

### Example 5: Increased peroxide accumulation in mouse lung fibroblasts (MLFs) derived from sesn^{-l-} mice.

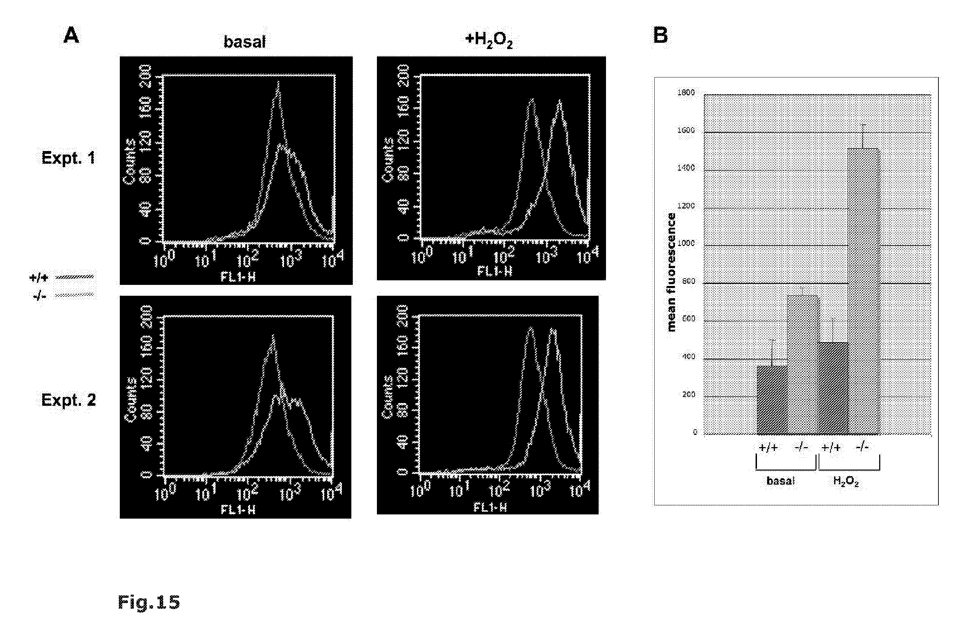

Recently published experiments in have shown that the inhibition of sesn2 expression by shRNA compromises the cell's ability to process ROS leading to ROS accumulation and oxidative stress (Budanov A. V., et al. Science. 304:596-600, 2004). In line with this, we expected antioxidant functions to be similarly compromised in cells derived from sesn2 knock out mice. To test this, we quantified basal and H₂O₂ induced ROS levels in MLFs using the dichlorodihydrofluorescein (DCF) fluorescence method. Figure 15 shows that *sesn*^{*-*/*-*} MLFs accumulated significantly more ROS than the corresponding wild type cells, regardless of pretreatment with 200 mM H₂O₂. The results are equivalent to those obtained in the previously described shRNA knock down experiments and are consistent with a sesn2 loss of function in the mutant MLFs.

### Sequence Listing, Free Text

| | | |
|---|---|---|
| SEQ ID NOs:1-9 | Primer | |
| SEQ ID NO: 10 | Gene trap vector pTβgeo plasmid Elements: | |
| | - En-2 Splice Acceptor | 2284-4163 |
| | - BGeo | 4164-8053 |
| | - SV40 pA | 8054-8496 |
| | - plasmid backbone | 8497-2283 |
| SEQ ID NOs:11-15 | Primer | |
| SEQ ID NO: 16 | Gene trap vector U3Cre Plasmid elements: | |
| | - LTRs 1-1475 and 3046-4520 | |
| | - Cre 3-1090 and 3074-4133 | |
| | - gag/env: 1475 - 3045. | |
| SEQ ID NOs: 17-24 | Primer | |

## Claims

1. A non-human animal model, which does not express one or more functional sestrins or expresses subotpimal levels of one or more sestrins and does not express latent transforming growth factor β binding protein 4 (Itbp4) or expresses subotpimal levels of Itbp4.

2. The animal model of claim 1, wherein
(i) the genome of the animal comprises a homozygous or a heterozygous disruption of a sestrin gene, preferably the disrupted sestrin gene being selected from sestrin 1, sestrin 2, and sestrin 3, and most preferably being sestrin 2; and/or
(ii) the genome of the animal comprises a homozygous or a heterozygous disruption of the ltbp4 gene.

3. The animal model of claim 2, wherein the disruption being generated by a mutation, preferably said mutation
(i) is an insertion, deletion or substitution mutation; and/or
(ii) is generated by gene targeting, gene trapping or chemical mutagenesis; and/or
(iii) has occurred in an exon, intron, regulatory region or splice site of the sestrin and Itbp4 genes; and/or
(iv) gives rise to the expression of a reporter gene; and/or
(v) has occurred in the 9^{th} intron of the sestrin 2 gene; and/or
(vi) has occurred in the 5^{th} intron of the Itpb4 gene.

4. The animal model of claim 2 or 3, wherein
(i) the sestrin 2 gene is disrupted in the 9^{th} intron by inserting a gene trap vector, preferably pT1βgeo (SEQ ID NO: 10); and/or
(ii) the ltbp4 gene is disrupted in the 5^{th} intron by inserting a gene trap vector, preferably U3Cre (SEQ ID NO: 16).

5. The animal model of any one of claims 1 to 4, wherein
(i) said non-human animal is a non-human mammal, preferably said non-human mammal is a rodent including mouse and rat; and/or
(ii) said animal exhibits pulmonary emphysema and cancer, preferably the cancer is a colon cancer.

6. A non-human animal model which does not express one or more functional sestrins or expresses subotpimal levels of one or more sestrins, preferably said animal model being as defined in claims 1 to 5.

7. A cell or tissue culture isolated from the animal models of claims 1 to 6.

8. The cell or tissue culture of claim 7, wherein the cell or tissue culture is from lung or colon.

9. A method for preparing the non-human animal model of claims 1 to 6, which comprises disrupting the sestrin and/or ltbp4 gene in a germ cell of the animal model.

10. The method of claim 9, wherein the non-human animal is a non-human mammal and the germ cell is an ES cell.

11. A method for selecting an agent for treating a symptom occurring in the non-human animal model of claim 1 comprising:
(i) applying one or more agents to be tested to said animal model,
(ii) determining whether one or more symptoms occurring in said animal model have changed as a result of application of said agent(s).

12. The method of claim 11, wherein the symptom is selected from a group consisting of cancer and pulmonary emphysema.

13. A method selecting an agent that interferes with ROS production and TGF-β signaling comprising:
(i) applying one or more agents to be tested to the to the cell or tissue culture of claim 7,
(ii) determining whether cellular ROS levels and TGF-β signaling have changed as a result of the application of said agents or agents.

14. The method of claim 13, wherein the cell or tissue culture is from lung or colon.

15. A method to analyze whether cancer and/or pulmonary emphysema is caused by differential Itbp4 and sestrin gene or protein expression or expression level or by a defect in the Itbp4 and sestrin gene comprising:
(i) characterizing the Itbp4 and sestrin gene or protein expression or expression level or Itbp4 and sestrin gene allele status of an individual having cancer or pulmonary emphysema,
(ii) characterizing the Itbp4 and sestrin gene or protein expression or expression level or Itbp4 and sestrin gene allele status of a control individual, a difference in the Itbp4 and sestrin gene or protein expression or expression level or Itbp4 and sestrin gene allele status indicating that cancer and/or pulmonary emphysema and/or cardiomyopathy is linked to differential Itbp4 and sestrin gene or protein expression or expression level or a defect in the Itbp4 and sestrin genes.

16. A method for diagnosing cancer and/or pulmonary emphysema and/or cardiomyopathy comprising:
(i) characterizing the Itbp4 and sestrin gene or protein expression or expression level or ltbp4 and sestrin gene allele status of an individual,
(ii) characterizing the ltbp4 and sestrin gene or protein expression or expression level or Itbp4 and sestrin gene allele status of a control individual, a difference in the LTBP-4 gene or protein expression or expression level or Itbp4 and sestrin gene allele status indicating the presence of cancer and/or pulmonary emphysema and/or cardiomyopathy in said individual.

17. The method of claim 15 or 16, wherein
(i) the ltbp4 and sestrin expression or expression level is detected by RT-PCR, Northern analysis, microarray analysis or antibodies directed to ltbp4 and sestrin proteins; and/or
(ii) the Itbp4 and sestrin gene expression or expression level is detected by RT-PCR, Northern analysis, microarray analysis or antibodies directed to ltbp4 and sestrin proteins; and/or
(iii) the Itbp4 and sestrin gene allele status is detected by mutation screening.

18. A kit for diagnosing cancer and/or pulmonary emphysema and/or cardiomyopathy comprising means to detect the Itbp4 and sestrin gene or protein expression or expression level or Itbp4 and sestrin gene allele status of an individual and control.


SEQUENCE LISTING

<110>  FrankGen Biotechnologie AG

<120>  Animal Model for the Selection and Validation of Agents Active
       Against Pulmonary Emphysema and Colorectal Cancer

<130>  062122ep/JH

<160>  24

<170>  PatentIn version 3.3

<210>  1
<211>  492
<212>  PRT
<213>  Mus musculus

<400>  1

Met Arg Leu Ala Ala Ala Ser Asn Glu Ala Tyr Ala Ala Ser Leu Ala
1               5                   10                  15

Val Ser Glu Leu Leu Ser Cys His Gln Cys Gly Gly Asp Arg Gly Gln
            20                  25                  30

Asp Glu Glu Leu Gly Ile Arg Ile Pro Arg Pro Leu Gly His Gly Pro
        35                  40                  45

Ser Arg Phe Ile Pro Glu Lys Glu Met Leu Gln Val Gly Ser Glu Asp
    50                  55                  60

Ala Gln Met His Ala Leu Phe Ala Asp Ser Phe Ala Ala Leu Gly Arg
65                  70                  75                  80

Leu Asp Asn Ile Thr Leu Val Met Val Phe His Pro Gln Tyr Leu Glu
                85                  90                  95

Ser Phe Leu Lys Thr Gln His Tyr Leu Leu Gln Met Asp Gly Pro Leu
            100                 105                 110

Pro Leu His Tyr Arg His Tyr Ile Gly Ile Met Ala Ala Ala Arg His
        115                 120                 125

Gln Cys Ser Tyr Leu Val Asn Leu His Val Ser Asp Phe Leu His Val
    130                 135                 140

Gly Gly Asp Pro Lys Trp Leu Asn Gly Leu Glu Asn Ala Pro Gln Lys
145                 150                 155                 160

Leu Gln Asn Leu Gly Glu Leu Asn Lys Val Leu Ala His Arg Pro Trp
                165                 170                 175

Leu Ile Thr Lys Glu His Ile Glu Gly Leu Leu Lys Ala Glu Glu His
            180                 185                 190

Ser Trp Ser Leu Ala Glu Leu Val His Ala Val Val Leu Leu Thr His
        195                 200                 205

Tyr His Ser Leu Ala Ser Phe Thr Phe Gly Cys Gly Ile Ser Pro Glu
    210                 215                 220

Ile His Cys Asp Gly Gly His Thr Phe Arg Pro Pro Ser Val Ser Asn
225                 230                 235                 240

Tyr Cys Ile Cys Asp Ile Thr Asn Gly Asn His Ser Val Asp Glu Met
                245                 250                 255

Gln Val Asn Ser Ala Gly Asn Ala Ser Val Ser Asp Ser Phe Phe Glu
            260                 265                 270

Val Glu Ala Leu Met Glu Lys Met Arg Gln Leu Gln Glu Cys Arg Glu
        275                 280                 285

Glu Glu Glu Ala Ser Gln Glu Glu Met Ala Ser Arg Phe Glu Met Glu
    290                 295                 300

Lys Arg Glu Ser Met Phe Val Phe Ser Ser Asp Asp Asp Glu Val Thr
305                 310                 315                 320

Pro Ala Arg Asp Val Ser Arg His Phe Glu Asp Thr Ser Tyr Gly Tyr
                325                 330                 335

Lys Asp Phe Ser Arg His Gly Met His Val Pro Thr Phe Arg Val Gln
            340                 345                 350

Asp Tyr Cys Trp Glu Asp His Gly Tyr Ser Leu Val Asn Arg Leu Tyr
        355                 360                 365

Pro Asp Val Gly Gln Leu Ile Asp Glu Lys Phe His Ile Pro Tyr Asn
    370                 375                 380

Leu Thr Tyr Asn Thr Met Ala Met His Lys Asp Val Asp Thr Ser Met
385                 390                 395                 400

Leu Arg Arg Ala Ile Trp Asn Tyr Ile His Cys Met Phe Gly Ile Arg
                405                 410                 415

Tyr Asp Asp Tyr Asp Tyr Gly Glu Ile Asn Gln Leu Leu Asp Arg Ser
            420                 425                 430

Phe Lys Val Tyr Ile Lys Thr Val Val Cys Thr Pro Glu Lys Val Thr
        435                 440                 445

Lys Arg Met Tyr Asp Ser Phe Trp Arg Gln Phe Lys His Ser Glu Lys
    450                 455                 460

Val His Val Asn Leu Leu Leu Ile Glu Ala Arg Met Gln Ala Glu Leu
465                 470                 475                 480

Leu Tyr Ala Leu Arg Ala Ile Thr Arg Tyr Met Thr
                485                 490

<210>  2
<211>  492
<212>  PRT
<213>  Mus musculus

<400>  2

Met Asn Arg Gly Gly Ser Ser Ala Ser Ala Ser Ala Asn Tyr Leu Leu
1               5                   10                  15

Cys Thr Asn Cys Arg Lys Val Leu Arg Lys Asp Lys Arg Ile Arg Val
            20                  25                  30

Ser Gln Pro Leu Thr Arg Gly Pro Ser Ala Phe Ile Pro Glu Lys Glu
        35                  40                  45

Val Val Gln Ala Asn Thr Ala Asp Glu Arg Thr Asn Phe Leu Val Glu
    50                  55                  60

Glu Tyr Ser Thr Ser Gly Arg Leu Asp Asn Ile Thr Gln Val Met Ser
65                  70                  75                  80

Leu His Thr Gln Tyr Leu Glu Ser Phe Leu Arg Ser Gln Phe Tyr Met
                85                  90                  95

Leu Arg Met Asp Gly Pro Leu Pro Leu Pro Asp Arg His Tyr Ile Ala
            100                 105                 110

Ile Met Ala Ala Ala Arg His Gln Cys Ser Tyr Leu Ile Asn Met His
        115                 120                 125

Val Asp Glu Phe Leu Lys Thr Gly Gly Ile Ala Glu Trp Leu Asn Gly
    130                 135                 140

Leu Glu Tyr Val Pro Gln Arg Leu Arg Asn Leu Asn Glu Ile Asn Lys
145                 150                 155                 160

Leu Leu Ala His Arg Pro Trp Leu Ile Thr Lys Glu His Ile Gln Lys
                165                 170                 175

Leu Val Lys Thr Gly Glu Asn Asn Trp Ser Leu Pro Glu Leu Val His
            180                 185                 190

Ala Val Val Leu Leu Ala His Tyr His Ala Leu Ala Ser Phe Val Phe
        195                 200                 205

Gly Ser Gly Ile Asn Pro Glu Arg Asp Pro Gly Ile Ala Asn Gly Phe
    210                 215                 220

Arg Leu Ile Ser Val Ser Ser Phe Cys Val Cys Asp Leu Ala Asn Asp
225                 230                 235                 240

Asn Ser Ile Glu Asn Thr Ser Leu Ala Gly Ser Asn Phe Gly Ile Val
                245                 250                 255

Asp Ser Leu Gly Glu Leu Glu Ala Leu Met Glu Arg Met Lys Arg Leu
            260                 265                 270

Gln Glu Asp Arg Glu Asp Asp Glu Thr Thr Arg Glu Glu Met Thr Thr
        275                 280                 285

Arg Phe Glu Lys Glu Lys Lys Glu Ser Leu Phe Val Val Pro Gly Glu
    290                 295                 300

Thr Leu His Ala Phe Pro His Ser Asp Phe Glu Asp Asp Val Ile Val
305                 310                 315                 320

Thr Ala Asp Val Ser Arg Tyr Ile Glu Asp Pro Ser Phe Gly Tyr Glu
                325                 330                 335

Asp Phe Ala Arg Arg Gly Glu Glu His Leu Pro Thr Phe Arg Ala Gln
            340                 345                 350

Asp Tyr Thr Trp Glu Asn His Gly Phe Ser Leu Val Asn Arg Leu Tyr
        355                 360                 365

Ser Asp Ile Gly His Leu Leu Asp Glu Lys Phe Arg Met Val Tyr Asn
    370                 375                 380

Leu Thr Tyr Asn Thr Met Ala Thr His Glu Asp Val Asp Thr Thr Thr

385                 390                 395                 400

Leu Arg Arg Ala Leu Phe Asn Tyr Val His Cys Met Phe Gly Ile Arg
                405                 410                 415

Tyr Asp Asp Tyr Asp Tyr Gly Glu Val Asn Gln Leu Leu Glu Arg Ser
            420                 425                 430

Leu Lys Val Tyr Ile Lys Thr Val Thr Cys Tyr Pro Glu Arg Thr Thr
        435                 440                 445

Lys Arg Met Tyr Asp Ser Tyr Trp Arg Gln Phe Thr His Ser Glu Lys
    450                 455                 460

Val His Val Asn Leu Leu Leu Met Glu Ala Arg Met Gln Ala Glu Leu
465                 470                 475                 480

Leu Tyr Ala Leu Arg Ala Ile Thr Arg His Leu Thr
                485                 490

<210>  3
<211>  480
<212>  PRT
<213>  Mus musculus

<400>  3

Met Ile Val Ala Asp Ser Glu Cys His Ser Glu Ile Lys Gly Tyr Leu
1               5                   10                  15

Pro Phe Thr Arg Gly Gly Val Ala Gly Pro Glu Thr Arg Glu Glu His
            20                  25                  30

Arg Glu Gly Gln Ala Arg Arg Gly Ser Arg Gly Pro Ser Ala Phe Ile
        35                  40                  45

Pro Val Glu Glu Ile Leu Arg Glu Gly Ala Glu Ser Leu Glu Gln His
    50                  55                  60

Leu Gly Leu Glu Ala Leu Met Ser Ser Gly Arg Val Asp Asn Leu Ala
65                  70                  75                  80

Val Val Met Gly Leu His Pro Asp Tyr Leu Ser Ser Phe Trp Arg Leu
                85                  90                  95

His Tyr Leu Leu Leu His Thr Asp Gly Pro Leu Ala Ser Ser Trp Arg
            100                 105                 110

His Tyr Ile Ala Ile Met Ala Ala Ala Arg His Gln Cys Ser Tyr Leu
        115                 120                 125

Val Gly Ser His Met Thr Glu Phe Leu Gln Thr Gly Gly Asp Pro Glu
    130                 135                 140

Trp Leu Leu Gly Leu His Arg Ala Pro Glu Lys Leu Arg Lys Leu Ser
145                 150                 155                 160

Glu Val Asn Lys Leu Leu Ala His Arg Pro Trp Leu Ile Thr Lys Glu
                165                 170                 175

His Ile Gln Ala Leu Leu Lys Thr Gly Glu His Ser Trp Ser Leu Ala
            180                 185                 190

Glu Leu Ile Gln Ala Leu Val Leu Leu Thr His Cys His Ser Leu Ala
        195                 200                 205

Ser Phe Val Phe Gly Cys Gly Ile Leu Pro Glu Gly Asp Ala Glu Gly
    210                 215                 220

Ser Pro Ala Ser Gln Ala Pro Ser Pro Pro Ser Glu Gln Gly Thr Pro
225                 230                 235                 240

Pro Ser Gly Asp Pro Leu Asn Asn Ser Gly Gly Phe Glu Ala Ala Arg
                245                 250                 255

Asp Val Glu Ala Leu Met Glu Arg Met Arg Gln Leu Gln Glu Ser Leu
            260                 265                 270

Leu Arg Asp Glu Gly Ala Ser Gln Glu Glu Met Glu Asn Arg Phe Glu
        275                 280                 285

Leu Glu Lys Ser Glu Ser Leu Leu Val Thr Pro Ser Ala Asp Ile Leu
    290                 295                 300

Glu Pro Ser Pro His Pro Asp Ile Leu Cys Phe Val Glu Asp Pro Ala
305                 310                 315                 320

Phe Gly Tyr Glu Asp Phe Thr Arg Arg Gly Thr Gln Ala Pro Pro Thr
                325                 330                 335

Phe Arg Ala Gln Asp Tyr Thr Trp Glu Asp His Gly Tyr Ser Leu Ile
            340                 345                 350

Gln Arg Leu Tyr Pro Glu Gly Gly Gln Leu Leu Asp Glu Lys Phe Gln
        355                 360                 365

Val Ala Cys Ser Leu Thr Tyr Asn Thr Ile Ala Met His Ser Gly Val
    370                 375                 380

Asp Thr Ser Met Leu Arg Arg Ala Ile Trp Asn Tyr Ile His Cys Val
385                 390                 395                 400

Phe Gly Ile Arg Tyr Asp Asp Tyr Asp Tyr Gly Glu Val Asn Gln Leu
                405                 410                 415

Leu Glu Arg Asn Leu Lys Ile Tyr Ile Lys Thr Val Ala Cys Tyr Pro
            420                 425                 430

Glu Lys Thr Thr Arg Arg Met Tyr Asn Leu Phe Trp Arg His Phe Arg
        435                 440                 445

His Ser Glu Lys Val His Val Asn Leu Leu Leu Leu Glu Ala Arg Met
    450                 455                 460

Gln Ala Ala Leu Leu Tyr Ala Leu Arg Ala Ile Thr Arg Tyr Met Thr
465                 470                 475                 480

<210>  4
<211>  48
<212>  DNA
<213>  Artificial

<220>
<223>  primer

<220>
<221>  misc_feature
<222>  (36)..(47)
<223>  n= inosine

<400>  4
ctactactac taggccacgc gtcgactagt acgggnnggg nngggnng                    48

<210>  5
<211>  22
<212>  DNA
<213>  Artificial

<220>
<223>  primer

<400>  5
gccagggttt tcccagtcac ga                                                22

<210>  6
<211>  32
<212>  DNA
<213>  Artificial

<220>
<223>  primer

<400>  6
ctactactac taggccacgc gtcgactagt ac                                    32


<210>  7
<211>  38
<212>  DNA
<213>  Artificial

<220>

<223>  primer

<400>  7
tgtaaaacga cggccagtgt gaaggctgtg cgaggccg                              38


<210>  8
<211>  21
<212>  DNA
<213>  Artificial

<220>
<223>  primer

<400>  8
cctggaacgg aacctcaaaa t                                                21


<210>  9
<211>  20
<212>  DNA
<213>  Artificial

<220>
<223>  primer

<400>  9
gggcttcaag gagcagcaag                                                  20


<210>  10
<211>  8950
<212>  DNA
<213>  Artificial

<220>
<223>  gene trap vector pTbetageo

<400>  10
tgtgagcaaa aggccagcaa aaggccagga accgtaaaaa ggccgcgttg ctggcgtttt      60

tccataggct ccgcccccct gacgagcatc acaaaaatcg acgctcaagt cagaggtggc     120

gaaacccgac aggactataa agataccagg cgtttccccc tggaagctcc ctcgtgcgct     180

ctcctgttcc gaccctgccg cttaccggat acctgtccgc ctttctccct tcgggaagcg     240

tggcgctttc tcatagctca cgctgtaggt atctcagttc ggtgtaggtc gttcgctcca     300

agctgggctg tgtgcacgaa ccccccgttc agcccgaccg ctgcgcctta tccggtaact     360

atcgtcttga gtccaacccg gtaagacacg acttatcgcc actggcagca gccactggta     420

acaggattag cagagcgagg tatgtaggcg gtgctacaga gttcttgaag tggtggccta 480

actacggcta cactagaaga acagtatttg gtatctgcgc tctgctgaag ccagttacct 540

tcggaaaaag agttggtagc tcttgatccg gcaaacaaac caccgctggt agcggtggtt 600

tttttgtttg caagcagcag attacgcgca gaaaaaaagg atctcaagaa gatcctttga 660

tcttttctac ggggtctgac gctcagtgga acgaaaactc acgttaaggg attttggtca 720

tgagattatc aaaaaggatc ttcacctaga tccttttaaa ttaaaaatga agttttaaat 780

caatctaaag tatatatgag taaacttggt ctgacagtta ccaatgctta atcagtgagg 840

cacctatctc agcgatctgt ctatttcgtt catccatagt tgcctgactc cccgtcgtgt 900

agataactac gatacgggag ggcttaccat ctggccccag tgctgcaatg ataccgcgag 960

acccacgctc accggctcca gatttatcag caataaacca gccagccgga agggccgagc 1020

gcagaagtgg tcctgcaact ttatccgcct ccatccagtc tattaattgt tgccgggaag 1080

ctagagtaag tagttcgcca gttaatagtt tgcgcaacgt tgttgccatt gctacaggca 1140

tcgtggtgtc acgctcgtcg tttggtatgg cttcattcag ctccggttcc caacgatcaa 1200

ggcgagttac atgatccccc atgttgtgca aaaaagcggt tagctccttc ggtcctccga 1260

tcgttgtcag aagtaagttg gccgcagtgt tatcactcat ggttatggca gcactgcata 1320

attctcttac tgtcatgcca tccgtaagat gcttttctgt gactggtgag tactcaacca 1380

agtcattctg agaatagtgt atgcggcgac cgagttgctc ttgcccggcg tcaatacggg 1440

ataataccgc gccacatagc agaactttaa aagtgctcat cattggaaaa cgttcttcgg 1500

ggcgaaaact ctcaaggatc ttaccgctgt tgagatccag ttcgatgtaa cccactcgtg 1560

cacccaactg atcttcagca tcttttactt tcaccagcgt ttctgggtga gcaaaaacag 1620

gaaggcaaaa tgccgcaaaa aagggaataa gggcgacacg gaaatgttga atactcatac 1680

tcttcctttt tcaatattat tgaagcattt atcagggtta ttgtctcatg agcggataca 1740

tatttgaatg tatttagaaa aataaacaaa taggggttcc gcgcacattt ccccgaaaag 1800

tgccacctga cgtctaagaa accattatta tcatgacatt aacctataaa aataggcgta 1860

tcacgaggcc ctttcgtctc gcgcgtttcg gtgatgacgg tgaaaacctc tgacacatgc 1920

agctcccgga gacggtcaca gcttgtctgt aagcggatgc cgggagcaga caagcccgtc 1980

agggcgcgtc agcgggtgtt ggcgggtgtc ggggctggct taactatgcg gcatcagagc 2040

agattgtact gagagtgcac catatgcggt gtgaaatacc gcacagatgc gtaaggagaa 2100

aataccgcat caggcgccat tcgccattca ggctgcgcaa ctgttgggaa gggcgatcgg 2160

tgcgggcctc ttcgctatta cgccagctgg cgaaaggggg atgtgctgca aggcgattaa 2220

gttgggtaac gccagggttt tcccagtcac gacgttgtaa aacgacggcc agtgccaagc 2280

ttggaattca tgggaagagg aaccgaaagt atgtttttca gatgttcttt ctcagaaata 2340
ggagtttgcg gaggttggag tgtgtgttgt aggacacgaa ccccagggtg gaggagactg 2400
gaggacagag ccctctttcc cagggaggga aggaggagag tttgagatcc gctccggaag 2460
tcggggttca ggtttgagca ggccaggcct ctcccgtggt ctcgccctct tgtcctagaa 2520
gcctcactgg ccaggtgtaa gccaggtcgt gggtgccgag ccctgctccc tcatcctcag 2580
catggatgtg aagaggactg tatggcgtgc gggtgtgtgt gaccgtgggt acacttaaaa 2640
caccgggttt tggatctgca ctgtcccgga tgtcctctgg tgctcaaaga cccttttggg 2700
tttgcccttt ggtaagagcg ccgggatcta cttgtctgga ggccagggag tcctcagccg 2760
aggcttgccg cccctgactg cactgcactg agtagtggat gggagagtct ggtaccgcac 2820
tgccggtttc ctccaccatc cccgcagcgc agggcagtgc attccgtcct ggctgcgaag 2880
ggggatggtc gggccttctc cagcctcttc cgcttctagc ggaaggggcc ttgatggaag 2940
ggcccgcatg tctccaaagt tgattcatgc ttcttgcaca gagaaagacc agaaagaagg 3000
tctcaagttt tagccggtag cccggatggc ctttcctgca cggcaccata tgaaccttgt 3060
gaccctgaca aagagacccc tctaacccaa gacccctacc acttaccctt tccctttgaa 3120
ggctttccca caccaccctc cacacttgcc ccaaacactg ccaactatgt aggaggaagg 3180
ggttgggact aacagaagaa cccgttgtgg ggaagctgtt gggagggtca ctttatgttc 3240
ttgcccaagg tcagttgggt ggcctgcttc tgatgaggtg gtcccaaggt ctggggtaga 3300
aggtgagagg gacaggccac caaggtcagc cccccccccc tatcccatag gagccaggtc 3360
cctctcctgg acaggaagac tgaaggggag atgccagaga ctcagtgaag cctggggtac 3420
cctattggag tccttcaagg aaacaaactt ggcctcacca ggcctcagcc ttggctcctc 3480
ctgggaactc tactgccctt gggatcccct tgtagttgtg ggttacatag gaagggggac 3540
gggattcccc ttgactggct agcctactct tttcttcagt cttctccatc tcctctcacc 3600
tgtctctcga ccctttccct aggatagact tggaaaaaga taaggggaga aaacaaatgc 3660
aaacgaggcc agaaagattt tggctgggca ttccttccgc tagcttttat tgggatcccc 3720
tagtttgtga taggcctttt agctacatct gccaatccat ctcattttca cacacacaca 3780
caccactttc cttctggtca gtgggcacat gtccagcctc aagtttatat caccaccccc 3840
aatgcccaac acttgtatgg ccttgggcgg gtcatccccc cccccacccc cagtatctgc 3900
aacctcaagc tagcttgggt gcgttggttg tggataagta gctagactcc agcaaccagt 3960
aacctctgcc ctttctcctc catgacaacc aggtcccagg tcccgaaaac caaagaagaa 4020
gaaccctaac aaagaggaca agcggcctcg cacagccttc actgctgagc agctccagag 4080
gctcaaggct gagtttcaga ccaacaggta cctgacagag cagcggcgcc agagtctggc 4140
acaggagctc ggtacccgga agatctggac tctagaggat cccgtcgttt tacaacgtcg 4200

tgactgggaa aaccctggcg ttacccaact taatcgcctt gcagcacatc cccctttcgc 4260

cagctggcgt aatagcgaag aggcccgcac cgatcgccct tcccaacagt tgcgcagcct 4320

gaatggcgaa tggcgctttg cctggtttcc ggcaccagaa gcggtgccgg aaagctggct 4380

ggagtgcgat cttcctgagg ccgatactgt cgtcgtcccc tcaaactggc agatgcacgg 4440

ttacgatgcg cccatctaca ccaacgtgac ctatcccatt acggtcaatc cgccgtttgt 4500

tcccacggag aatccgacgg gttgttactc gctcacattt aatgttgatg aaagctggct 4560

acaggaaggc cagacgcgaa ttatttttga tggcgttaac tcggcgtttc atctgtggtg 4620

caacgggcgc tgggtcggtt acggccagga cagtcgtttg ccgtctgaat ttgacctgag 4680

cgcattttta cgcgccggag aaaaccgcct cgcggtgatg gtgctgcgct ggagtgacgg 4740

cagttatctg gaagatcagg atatgtggcg gatgagcggc attttccgtg acgtctcgtt 4800

gctgcataaa ccgactacac aaatcagcga tttccatgtt gccactcgct ttaatgatga 4860

tttcagccgc gctgtactgg aggctgaagt tcagatgtgc ggcgagttgc gtgactacct 4920

acgggtaaca gtttctttat ggcagggtga aacgcaggtc gccagcggca ccgcgccttt 4980

cggcggtgaa attatcgatg agcgtggtgg ttatgccgat cgcgtcacac tacgtctgaa 5040

cgtcgaaaac ccgaaactgt ggagcgccga aatcccgaat ctctatcgtg cggtggttga 5100

actgcacacc gccgacggca cgctgattga agcagaagcc tgcgatgtcg gtttccgcga 5160

ggtgcggatt gaaaatggtc tgctgctgct gaacggcaag ccgttgctga ttcgaggcgt 5220

taaccgtcac gagcatcatc ctctgcatgg tcaggtcatg gatgagcaga cgatggtgca 5280

ggatatcctg ctgatgaagc agaacaactt taacgccgtg cgctgttcgc attatccgaa 5340

ccatccgctg tggtacacgc tgtgcgaccg ctacggcctg tatgtggtgg atgaagccaa 5400

tattgaaacc cacggcatgg tgccaatgaa tcgtctgacc gatgatccgc gctggctacc 5460

ggcgatgagc gaacgcgtaa cgcgaatggt gcagcgcgat cgtaatcacc cgagtgtgat 5520

catctggtcg ctggggaatg aatcaggcca cggcgctaat cacgacgcgc tgtatcgctg 5580

gatcaaatct gtcgatcctt cccgcccggt gcagtatgaa ggcggcggag ccgacaccac 5640

ggccaccgat attatttgcc cgatgtacgc gcgcgtggat gaagaccagc ccttcccggc 5700

tgtgccgaaa tggtccatca aaaaatggct ttcgctacct ggagagacgc gcccgctgat 5760

cctttgcgaa tacgcccacg cgatgggtaa cagtcttggc ggtttcgcta aatactggca 5820

ggcgtttcgt cagtatcccc gtttacaggg cggcttcgtc tgggactggg tggatcagtc 5880

gctgattaaa tatgatgaaa acggcaaccc gtggtcggct tacggcggtg attttggcga 5940

tacgccgaac gatcgccagt tctgtatgaa cggtctggtc tttgccgacc gcacgccgca 6000

tccagcgctg acggaagcaa aacaccagca gcagtttttc cagttccgtt tatccgggca 6060

aaccatcgaa gtgaccagcg aatacctgtt ccgtcatagc gataacgagc tcctgcactg 6120

gatggtggcg ctggatggta agccgctggc aagcggtgaa gtgcctctgg atgtcgctcc 6180

acaaggtaaa cagttgattg aactgcctga actaccgcag ccggagagcg ccgggcaact 6240

ctggctcaca gtacgcgtag tgcaaccgaa cgcgaccgca tggtcagaag ccgggcacat 6300

cagcgcctgg cagcagtggc gtctggcgga aaacctcagt gtgacgctcc ccgccgcgtc 6360

ccacgccatc ccgcatctga ccaccagcga aatggatttt tgcatcgagc tgggtaataa 6420

gcgttggcaa tttaaccgcc agtcaggctt tctttcacag atgtggattg gcgataaaaa 6480

acaactgctg acgccgctgc gcgatcagtt cacccgtgca ccgctggata acgacattgg 6540

cgtaagtgaa gcgacccgca ttgaccctaa cgcctgggtc gaacgctgga aggcggcggg 6600

ccattaccag gccgaagcag cgttgttgca gtgcacggca gatacacttg ctgatgcggt 6660

gctgattacg accgctcacg cgtggcagca tcaggggaaa accttattta tcagccggaa 6720

aacctaccgg attgatggta gtggtcaaat ggcgattacc gttgatgttg aagtggcgag 6780

cgatacaccg catccggcgc ggattggcct gaactgccag ctggcgcagg tagcagagcg 6840

ggtaaactgg ctcggattag ggccgcaaga aaactatccc gaccgcctta ctgccgcctg 6900

ttttgaccgc tgggatctgc cattgtcaga catgtatacc ccgtacgtct tcccgagcga 6960

aaacggtctg cgctgcggga cgcgcgaatt gaattatggc ccacaccagt ggcgcggcga 7020

cttccagttc aacatcagcc gctacagtca acagcaactg atggaaacca gccatcgcca 7080

tctgctgcac gcggaagaag gcacatggct gaatatcgac ggtttccata tggggattgg 7140

tggcgacgac tcctggagcc cgtcagtatc ggcggaattc cagctgagcg ccggtcgcta 7200

ccattaccag ttggtctggt gtcaggggat cccccgggct gcagccaata tgggatcggc 7260

cattgaacaa gatggattgc acgcaggttc tccggccgct tgggtggaga ggctattcgg 7320

ctatgactgg gcacaacaga caatcggctg ctctgatgcc gccgtgttcc ggctgtcagc 7380

gcaggggcgc ccggttcttt ttgtcaagac cgacctgtcc ggtgccctga atgaactgca 7440

ggacgaggca gcgcggctat cgtggctggc cacgacgggc gttccttgcg cagctgtgct 7500

cgacgttgtc actgaagcgg gaagggactg gctgctattg ggcgaagtgc cggggcagga 7560

tctcctgtca tctcaccttg ctcctgccga gaaagtatcc atcatggctg atgcaatgcg 7620

gcggctgcat acgcttgatc cggctacctg cccattcgac caccaagcga aacatcgcat 7680

cgagcgagca cgtactcgga tggaagccgg tcttgtcgat caggatgatc tggacgaaga 7740

gcatcagggg ctcgcgccag ccgaactgtt cgccaggctc aaggcgcgca tgcccgacgg 7800

cgaggatctc gtcgtgaccc atggcgatgc ctgcttgccg aatatcatgg tggaaaatgg 7860

ccgcttttct ggattcatcg actgtggccg gctgggtgtg gcggaccgct atcaggacat 7920

agcgttggct acccgtgata ttgctgaaga gcttggcggc gaatgggctg accgcttcct 7980

cgtgctttac ggtatcgccg ctcccgattc gcagcgcatc gccttctatc gccttcttga    8040

cgagttcttc tgagcgggac tctggggttc gaaatgaccg accaagcgac gcccaacctg    8100

ccatcacgag atttcgattc caccgccgcc ttctatgaaa ggttgggctt cggaatcgtt    8160

ttccgggacg ccggctggat gatcctccag cgcggggatc tcatgctgga gttcttcgcc    8220

caccccccgg atctaagctc tagataagta atgatcataa tcagccatat cacatctgta    8280

gaggttttac ttgctttaaa aaacctccca cacctccccc tgaacctgaa acataaaatg    8340

aatgcaattg ttgttgttaa cttgtttatt gcagcttata atggttacaa ataaagcaat    8400

agcatcacaa atttcacaaa taaagcattt ttttcactgc attctagttg tggtttgtcc    8460

aaactcatca atgtatctta tcatgtctgg atccgggggt accgagctcg aattcgtaat    8520

catgtcatag ctgtttcctg tgtgaaattg ttatccgctc acaattccac acaacatacg    8580

agccggaagc ataaagtgta aagcctgggg tgcctaatga gtgagctaac tcacattaat    8640

tgcgttgcgc tcactgcccg ctttccagtc gggaaacctg tcgtgccagc tgcattaatg    8700

aatcggccaa cgcgcgggga gaggcggttt gcgtattggg cgctcttccg cttcctcgct    8760

cactgactcg ctgcgctcgg tcgttcggct gcggcgagcg gtatcagctc actcaaaggc    8820

ggtaatacgg ttatccacag aatcagggga taacgcagga aagaacatgt gagcaaaagg    8880

ccagcaaaag gccaggaacc gtaaaaaggc cgcgttgctg gcgtttttcc ataggctccg    8940

cccccctgac                                                          8950

<210>  11
<211>  20
<212>  DNA
<213>  Artificial

<220>
<223>  primer

<400>  11
cagccttgag cctctggagc                                               20

<210>  12
<211>  21
<212>  DNA
<213>  Artificial

<220>
<223>  primer

<400>  12
ctaccctgag aagacgaccc g                                             21

<210>  13
<211>  21
<212>  DNA

<213>  Artificial

<220>
<223>  primer

<400>  13
ctaccctgag aagacgaccc g                                                21


<210>  14
<211>  30
<212>  DNA
<213>  Artificial

<220>
<223>  primer

<400>  14
ggacaaatca aggttacaca gaaaaaagtc                                       30


<210>  15
<211>  20
<212>  DNA
<213>  Artificial

<220>
<223>  primer

<400>  15
cagccttgag cctctggagc                                                  20


<210>  16
<211>  4520
<212>  DNA
<213>  Artificial

<220>
<223>  gene trap vector U3Cre

<400>  16
aatgaaagac cccacctgta ggtttggcaa gctagcatgc ccaagaagaa gaggaaggtg      60

tccaatttac tgaccgtaca ccaaaatttg cctgcattac cggtcgatgc aacgagtgat     120

gaggttcgca agaacctgat ggacatgttc agggatcgcc aggcgttttc tgagcatacc     180

tggaaaatgc ttctgtccgt ttgccggtcg tgggcggcat ggtgcaagtt gaataaccgg     240

aaatggtttc ccgcagaacc tgaagatgtt cgcgattatc ttctatatct tcaggcgcgc     300

ggtctggcag taaaaactat ccagcaacat ttgggccagc taaacatgct tcatcgtcgg     360

tccgggctgc cacgaccaag tgacagcaat gctgtttcac tggttatgcg gcggatccga     420

aaagaaaacg ttgatgccgg tgaacgtgca aaacaggctc tagcgttcga acgcactgat     480

ttcgaccagg ttcgttcact catggaaaat agcgatcgct gccaggatat acgtaatctg     540

gcatttctgg ggattgctta taacaccctg ttacgtatag ccgaaattgc caggatcagg     600

gttaaagata tctcacgtac tgacggtggg agaatgttaa tccatattgg cagaacgaaa     660

acgctggtta gcaccgcagg tgtagagaag gcacttagcc tgggggtaac taaactggtc 720

gagcgatgga tttccgtctc tggtgtagct gatgatccga ataactacct gttttgccgg 780

gtcagaaaaa atggtgttgc cgcgccatct gccaccagcc agctatcaac tcgcgccctg 840

gaagggattt ttgaagcaac tcatcgattg atttacggcg ctaaggatga ctctggtcag 900

agatacctgg cctggtctgg acacagtgcc cgtgtcggag ccgcgcgaga tatggcccgc 960

gctggagttt caataccgga gatcatgcaa gctggtggct ggaccaatgt aaatattgtc 1020

atgaactata tccgtaacct ggatagtgaa acaggggcaa tggtgcgcct gctggaagat 1080

ggcgattagg ctagcttaag taacgccatt ttgcaaggca tggaaaaata cataactgag 1140

aatagagaag ttcagatcaa ggtcaggaac agatggaaca gctagagaac catcagatgt 1200

ttccagggtg ccccaaggac ctgaaatgac cctgtgcctt atttgaacta accaatcagt 1260

tcgcttctcg cttctgttcg cgcgcttctg ctccccgagc tcaataaaag agcccacaac 1320

ccctcactcg gggcgccagt cctccgattg actgagtcgc ccgggtaccc gtgtatccaa 1380

taaaccctct tgcagttgca tccgacttgt ggtctcgctg ttccttggga gggtctcctc 1440

tgagtgattg actacccgtc agcgggggtc tttcatttgg gggctcgtcc gggatcggga 1500

gacccctgcc cagggaccac cgacccacca ccgggaggta agctggccag caacttatct 1560

gtgtctgtcc gattgtctag tgtctatgac tgattttatg cgcctgcgtc ggtactagtt 1620

agctaactag ctctgtatct ggcggacccg tggtggaact gacgagttcg gaacacccgg 1680

ccgcaaccct gggagacgtc ccagggactt cgggggccgt ttttgtggcc cgacctgagt 1740

ccaaaaatcc cgatcgtttt ggactctttg gtgcaccccc cttagaggag ggatatgtgg 1800

ttctggtagg agacgagaac ctaaaacagt tcccgcctcc gtctgaattt ttgctttcgg 1860

tttgggaccg aagccgcgcc gcgcgtcttg tctgctgcag catcgttctg tgttgtctct 1920

gtctgactgt gtttctgtat ttgtctgaga atatgggcca gactgttacc actcccttaa 1980

gtttgacctt aggtcactgg aaagatgtcg agcggatcgc tcacaaccag tcggtagatg 2040

tcaagaagag acgttgggtt accttctgct ctgcagaatg gccaaccttt aacgtcggat 2100

ggccgcgaga cggcaccttt aaccgagacc tcatcaccca ggttaagatc aaggtctttt 2160

cacctggccc gcatggacac ccagaccagg tcccctacat cgtgacctgg gaagccttgg 2220

cttttgaccc ccctccctgg gtcaagccct ttgtacaccc taagcctccg cctcctcttc 2280

ctccatccgc cccgtctctc ccccttgaac ctcctcgttc gaccccgcct cgatcctccc 2340

tttatccagc cctcactcct tctctaggcg ccaaacctaa acctcaagtt ctttctgaca 2400

gtggggggcc gctcatcgac ctacttacag aagacccccc gccttatagg gacccaagac 2460

cacccccttc cgacagggac ggaaatggtg gagaagcgac ccctgcggga gaggcaccgg 2520

acccctcccc aattgcatct cgcctacgtg ggagacggga gccccctgtg gccgactcca 2580

ctacctcgca ggcattcccc ctccgcgcag gaggaaacgg acagcttcaa tactggccgt 2640

tctcctcttc tgacctttac aactggaaaa ataataaccc ttctttttct gaagatccag 2700

gtaaactgac agctctgatc gagtctgttc tcatcaccca tcagcccacc tgggacgact 2760

gtcagcagct gttggggact ctgctgaccg gagaagaaaa acaacgggtg ctcttagagg 2820

ctagaaaggc ggtgcggggc gatgatgggc gccccactca actgcccaat gaagtcgatg 2880

ccgcttttcc cctcgaggga tccatcgtag tccaatttgt taaagacagg atatcagtgg 2940

tccaggctct agttttgact caacaatatc accagctgaa gcctatagag tacgagccat 3000

agataaaata aaagatttta tttagtctcc agaaaaaggg gggaatgaaa gaccccacct 3060

gtaggtttgg caagctagca tgcccaagaa gaagaggaag gtgtccaatt tactgaccgt 3120

acaccaaaat ttgcctgcat taccggtcga tgcaacgagt gatgaggttc gcaagaacct 3180

gatggacatg ttcagggatc gccaggcgtt ttctgagcat acctggaaaa tgcttctgtc 3240

cgtttgccgg tcgtgggcgg catggtgcaa gttgaataac cggaaatggt ttcccgcaga 3300

acctgaagat gttcgcgatt atcttctata tcttcaggcg cgcggtctgg cagtaaaaac 3360

tatccagcaa catttgggcc agctaaacat gcttcatcgt cggtccgggc tgccacgacc 3420

aagtgacagc aatgctgttt cactggttat gcggcggatc cgaaaagaaa acgttgatgc 3480

cggtgaacgt gcaaaacagg ctctagcgtt cgaacgcact gatttcgacc aggttcgttc 3540

actcatggaa aatagcgatc gctgccagga tatacgtaat ctggcatttc tggggattgc 3600

ttataacacc ctgttacgta tagccgaaat tgccaggatc agggttaaag atatctcacg 3660

tactgacggt gggagaatgt taatccatat tggcagaacg aaaacgctgg ttagcaccgc 3720

aggtgtagag aaggcactta gcctgggggt aactaaactg gtcgagcgat ggatttccgt 3780

ctctggtgta gctgatgatc cgaataacta cctgttttgc cgggtcagaa aaaatggtgt 3840

tgccgcgcca tctgccacca gccagctatc aactcgcgcc ctggaaggga tttttgaagc 3900

aactcatcga ttgatttacg gcgctaagga tgactctggt cagagatacc tggcctggtc 3960

tggacacagt gcccgtgtcg gagccgcgcg agatatggcc cgcgctggag tttcaatacc 4020

ggagatcatg caagctggtg gctggaccaa tgtaaatatt gtcatgaact atatccgtaa 4080

cctggatagt gaaacagggg caatggtgcg cctgctggaa gatggcgatt aggctagctt 4140

aagtaacgcc attttgcaag gcatggaaaa atacataact gagaatagag aagttcagat 4200

caaggtcagg aacagatgga acagctagag aaccatcaga tgtttccagg gtgccccaag 4260

gacctgaaat gaccctgtgc cttatttgaa ctaaccaatc agttcgcttc tcgcttctgt 4320

tcgcgcgctt ctgctccccg agctcaataa aagagcccac aacccctcac tcggggcgcc 4380

agtcctccga ttgactgagt cgcccgggta cccgtgtatc caataaaccc tcttgcagtt 4440

gcatccgact tgtggtctcg ctgttccttg ggagggtctc ctctgagtga ttgactaccc 4500

gtcagcgggg gtctttcatt 4520

<210> 17

<211> 23
<212> DNA
<213> Artificial

<220>
<223> primer

<400> 17
ccaatcttgc ttctttgctg agc 23

<210> 18
<211> 22
<212> DNA
<213> Artificial

<220>
<223> primer

<400> 18
ggctcatgct tgaatgtttc ag 22

<210> 19
<211> 23
<212> DNA
<213> Artificial

<220>
<223> primer

<400> 19
ccaatcttgc ttctttgctg agc 23

<210> 20
<211> 20
<212> DNA
<213> Artificial

<220>
<223> primer

<400> 20
atcatgcaag ctggtggctg 20

<210> 21
<211> 21
<212> DNA
<213> Artificial

<220>
<223> primer

<400>  21
ctaccctgag aagacgaccc g                                                 21


<210>  22
<211>  30
<212>  DNA
<213>  Artificial

<220>
<223>  primer

<400>  22
ggacaaatca aggttacaca gaaaaaagtc                                        30


<210>  23
<211>  21
<212>  DNA
<213>  Artificial

<220>
<223>  primer

<400>  23
ctaccctgag aagacgaccc g                                                 21


<210>  24
<211>  20
<212>  DNA
<213>  Artificial

<220>
<223>  primer

<400>  24
cagccttgag cctctggagc                                                   20